# EUROPEAN PATENT SPECIFICATION

(11) **EP 1 137 761 B1**
(45) Date of publication and mention of the grant of the patent: **01.08.2007**
(21) Application number: 99957265.4
(22) Date of filing: 03.12.1999
(51) Int. Cl.: C12N 9/18

(54) **CUTINASE VARIANTS**
CUTINASE-VARIANTEN
VARIANTES DE CUTINASE

(30) Priority: 04.12.1998 DK 160498; 09.03.1999 DK 990330
(43) Date of publication of application: 04.10.2001
(73) Proprietor: Novozymes A/S, 2880 Bagsvaerd (DK)
(72) Inventor: ABO, Masanobu, Funabashi-shi Chiba-ken 274 (JP); FUKUYAMA, Shiro, Chiba-shi Chiba 211-0012 (JP); SVENDSEN, Allan, DK-3460 Birkerod (DK); MATSUI, Tomoko, Ichikawa Chiba 272-0026 (JP)
(74) Representative: Knudsen, Sten Lottrup
(86) International application number: PCT/DK1999/000678
(87) International publication number: WO 2000/034450

(56) References cited:
- WO-A1-90/09446
- WO-A1-94/14963
- WO-A1-94/14964
- WO-A1-97/04078
- SONIA LONGHI ET AL.: 'Dynamics of Fusarium solani Cutinase Investigated Through Structural Comparison Among Different Crystal Forms of Its Variants' PROTEINS: STRUCTURE, FUNCTION AND GENETICS vol. 26, 1996, pages 442 - 458, XP002926640

## Description

### FIELD OF THE INVENTION

The present invention relates to a cutinase variant, more particularly to a cutinase variant having improved thermostability. The invention also relates to a DNA sequence encoding the variant, a vector comprising the DNA sequence, a transformed host cell harboring the DNA sequence or the vector, to a method of producing the variant, and to use of the variant.

### BACKGROUND OF THE INVENTION

Cutinases are lipolytic enzymes capable of hydrolyzing the substrate cutin. Cutinases are known from various fungi (P.E. Kolattukudy in "Lipases", Ed. B. Borgström and H.L. Brockman, Elsevier 1984, 471-504). The amino acid sequence and the crystal structure of a cutinase of *Fusarium solani pisi* have been described (S. Longhi et al., Journal of Molecular Biology, 268 (4), 779-799 (1997)). The amino acid sequence of a cutinase from *Humicola insolens* has also been published (US 5,827,719).

A number of variants of the cutinase of *Fusarium solani pisi* have been published: WO 94/14963; WO 94/14964; Appl. Environm. Microbiol. 64, 2794-2799, 1998; Proteins: Structure, Function and Genetics 26, 442-458, 1996; J. of Computational Chemistry 17, 1783-1803, 1996; Protein Engineering 6, 157-165, 1993; Proteins: Structure, Function, and Genetics 33, 253-264, 1998; J. of Biotechnology 66, 11-26, 1998; Biochemistry 35, 398-410, 1996.

Fungal cutinases may be used in the enzymatic hydrolysis of cyclic oligomers of poly(ethylene terephthalate), e.g. in the finishing of yarn or fabric from poly(ethylene terephthalate) fibers (WO 97/27237). However, it is desirable to improve the thermostability of known fungal cutinases to allow a higher process temperature.

### SUMMARY OF THE INVENTION

The inventors have found certain variants of fungal cutinases having improved thermostability.

Accordingly, the invention provides a variant of a parent fungal cutinase
wherein:
a) the parent cutinase has an amino acid sequence which is at least 70 % homologous to the cutinase of *H. insolens* strain DSM 1800,
b) the variant has an amino acid sequence having 1-20 alterations compared to the parent cutinase,
c) the variant comprises substitution of a negative amino acid residue at a position corresponding to position E6, E10, E30, E47 D63, E82 and/or E179 by a neutral or positive amino acid or a substitution with Pro at a position corresponding to A14 or R51 in the parent cutinase,
d) the variant has hydrolytic activity towards cyclic tri(ethylene terephthalate) or terephthalic acid bis(2-hydroxyethyl)ester dibenzoate, and
e) the variant is more thermostable than the parent cutinase.

The invention also provides a DNA sequence encoding the variant, an expression vector comprising the DNA sequence, a transformed host cell harboring the DNA sequence or the expression vector, a method of producing the variant, processes using the variant and a detergent composition comprising the variant.

### BRIEF DESCRIPTION OF THE DRAWINGS

Fig. 1 gives the coordinates for the 3D structure of the cutinase of *H. insolens.*
Fig. 2 is a computer model showing the three-dimensional structures of the cutinases from *F. solani pisi* (left) and *H. insolens* (right). Different colors have been used to identify the N-terminal amino acid and zones of 12 Å and 17 Å diameter around this.
Figs. 3-6 illustrate the hydrolysis of c3ET. Details are given in the Examples.

### DETAILED DESCRIPTION OF THE INVENTION

### Fungal cutinase

The parent cutinase is a fungal cutinase, such as a filamentous fungal cutinase, e.g. native to a strain of *Humicola* or *Fusarium,* specifically *H. insolens* or *F*. *solani pisi,* more specifically *H. insolens* strain DSM 1800.

The amino acid sequence of the cutinase of *H. insolens* strain DSM 1800 and the DNA sequence encoding it are shown as SEQ ID NO: 2 and SEQ ID NO: 1 of US 5,827,719. The numbering system used herein for the *H. insolens* cutinase is based on the mature peptide, as shown in said SEQ ID NO: 2.

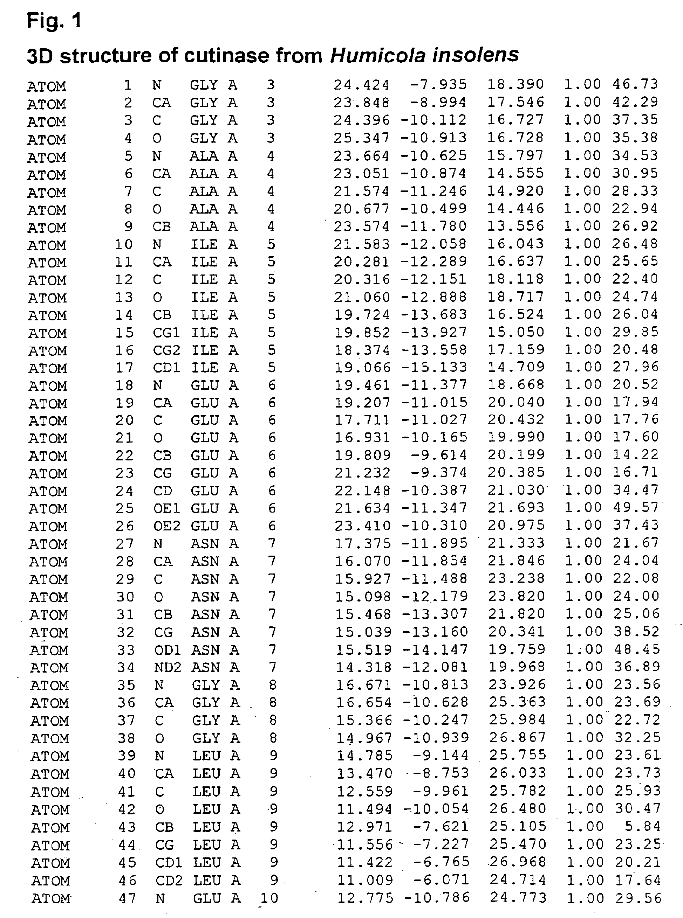

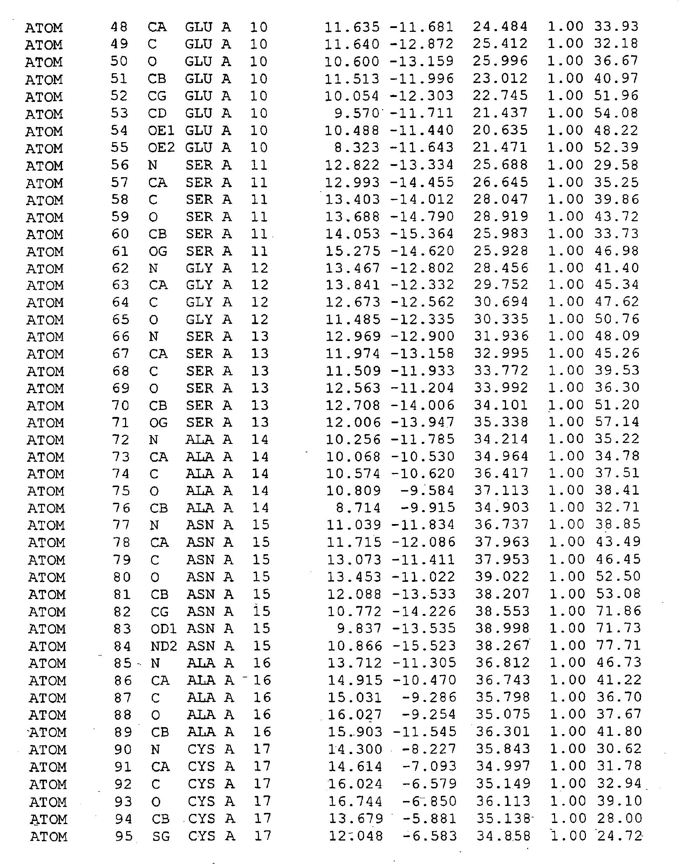

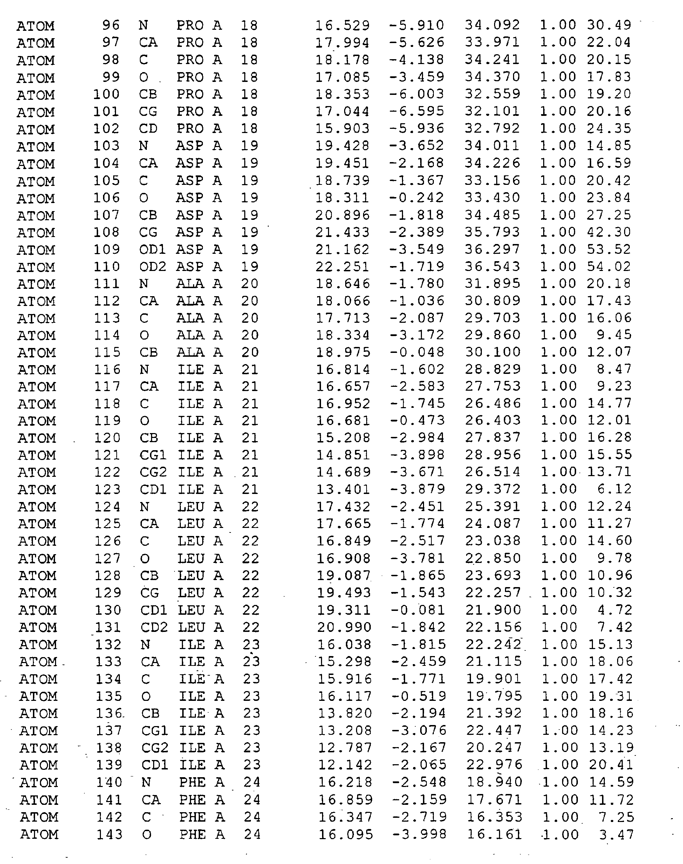

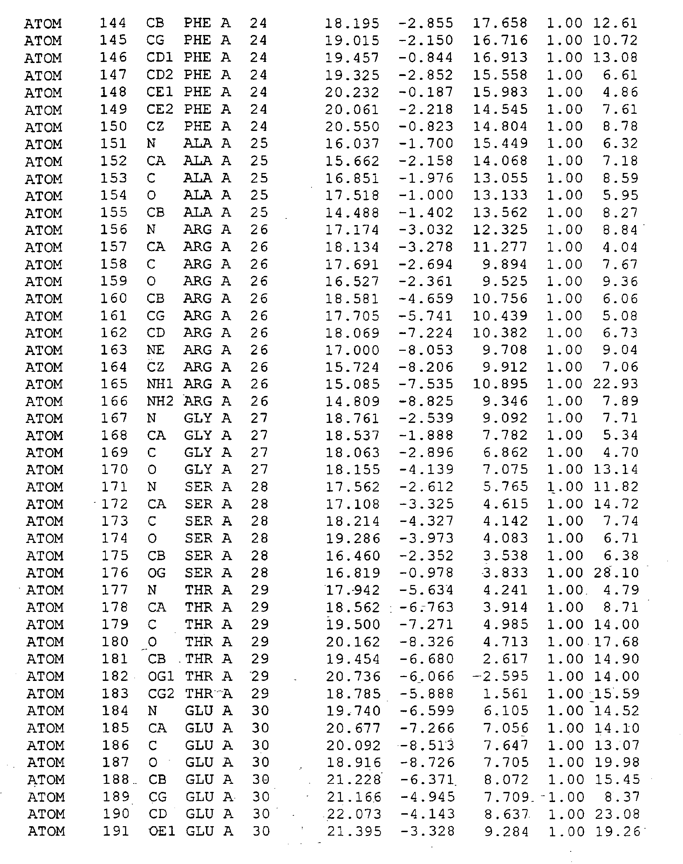

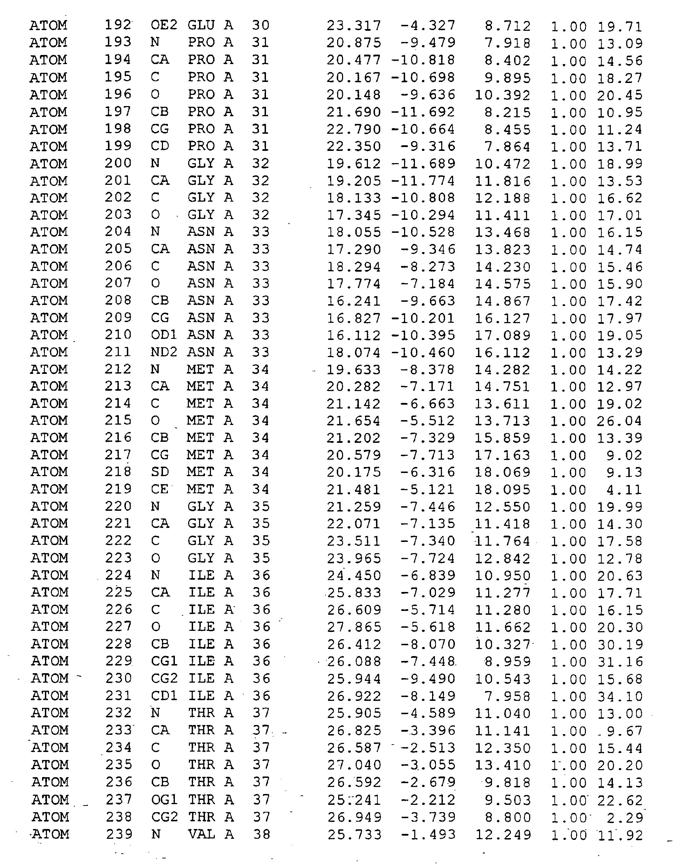

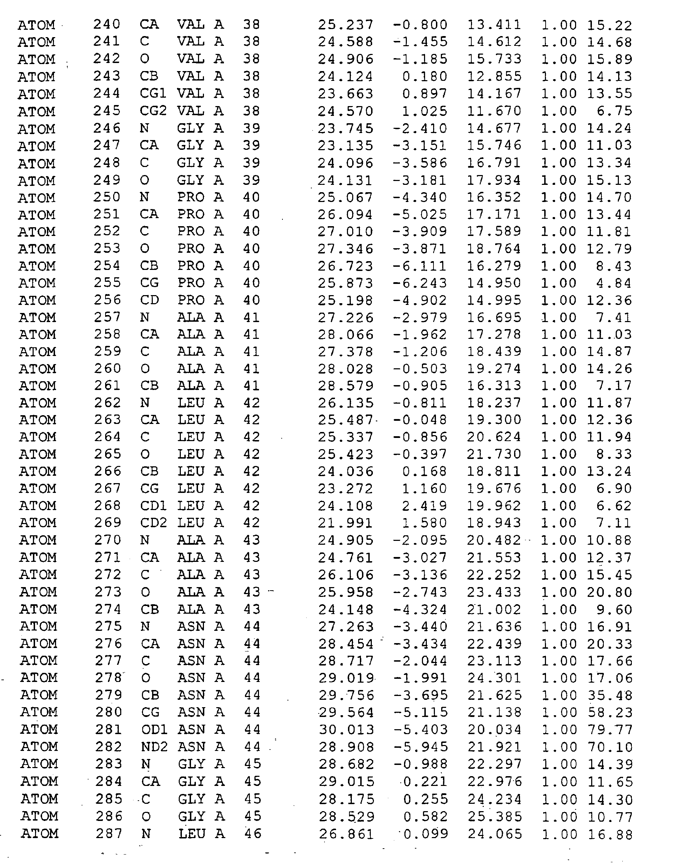

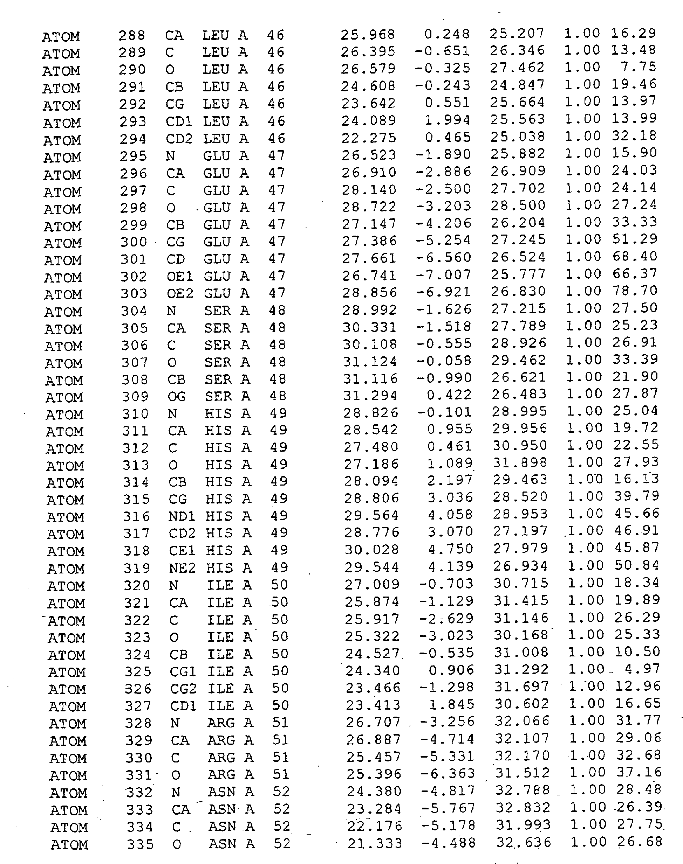

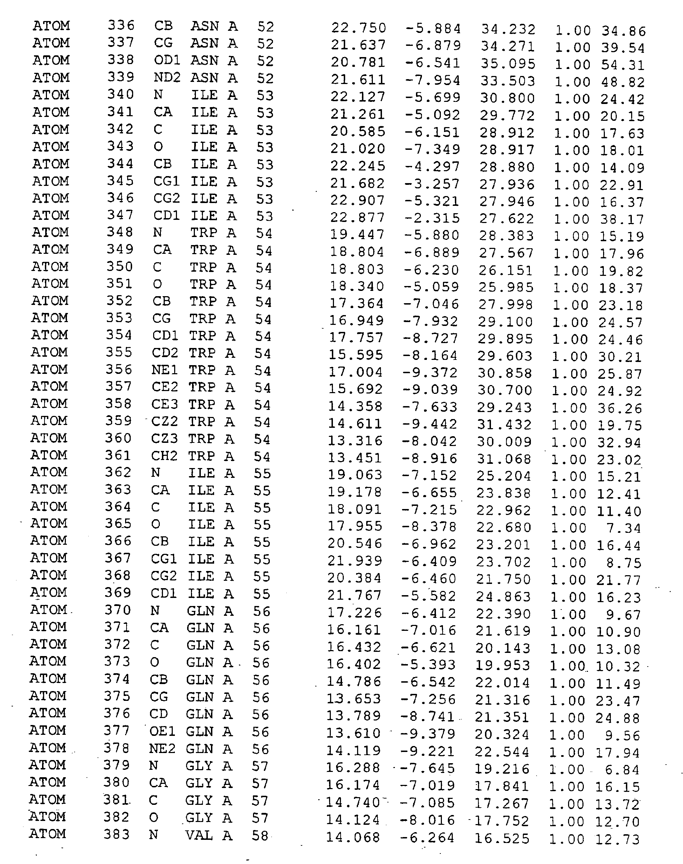

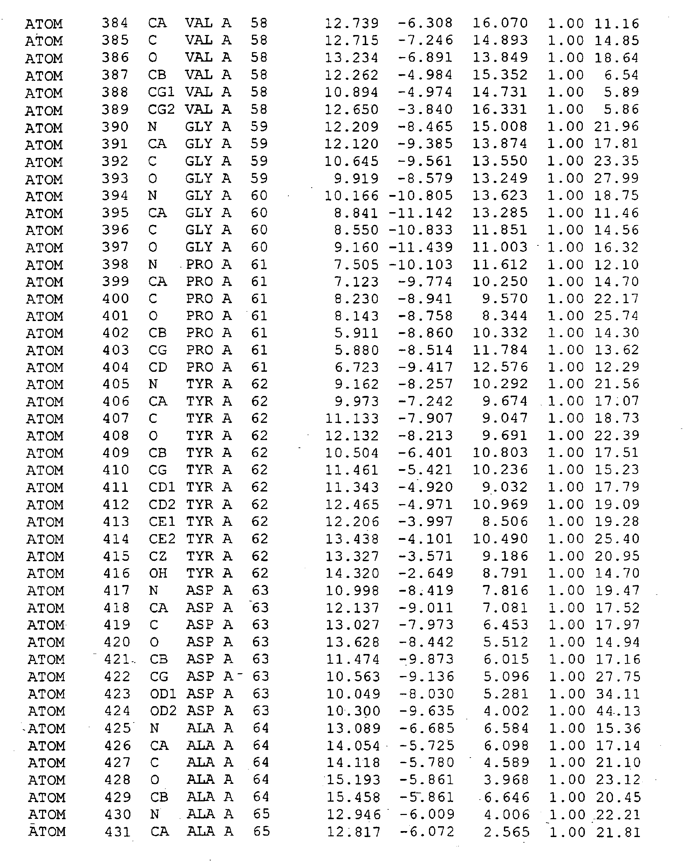

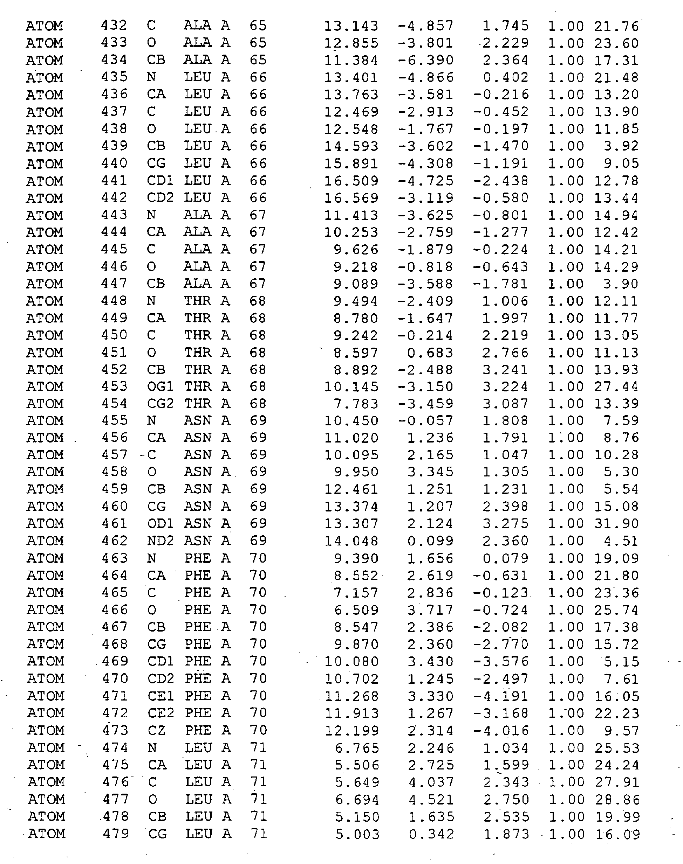

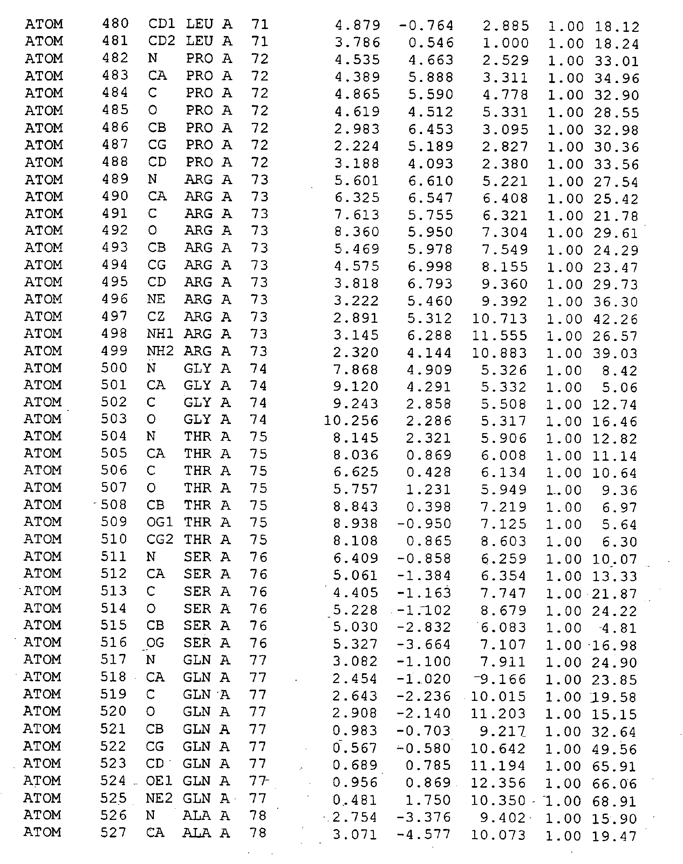

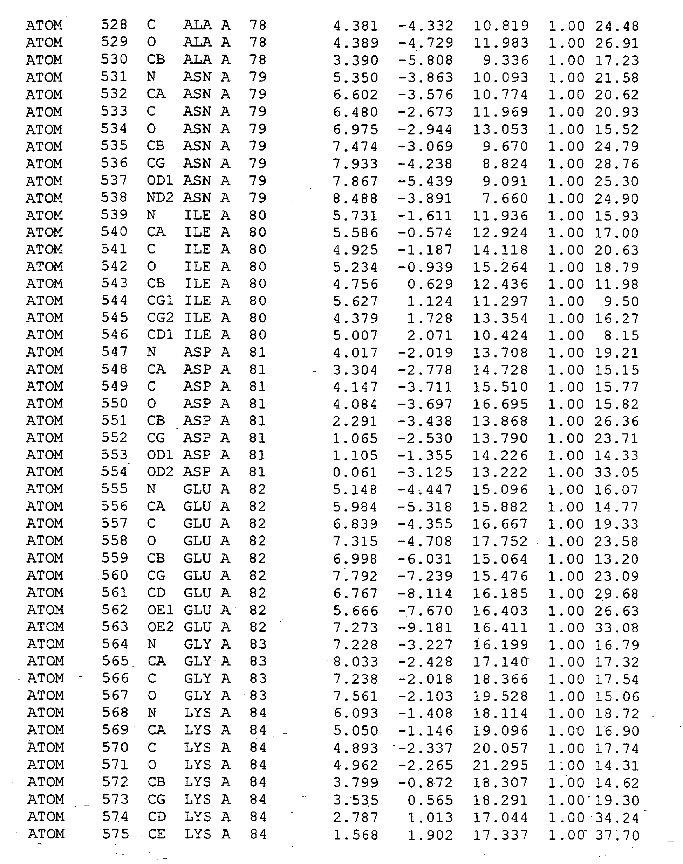

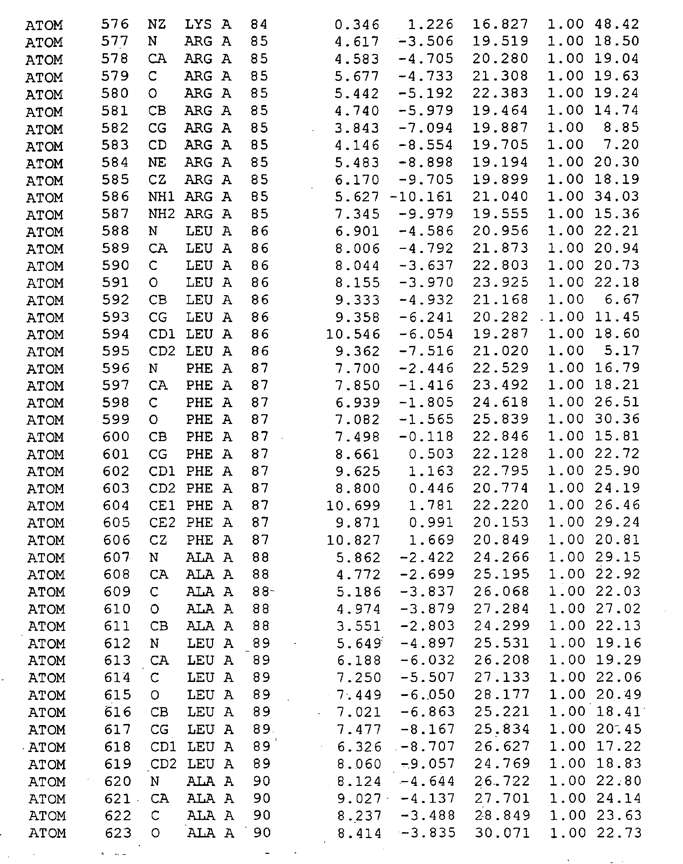

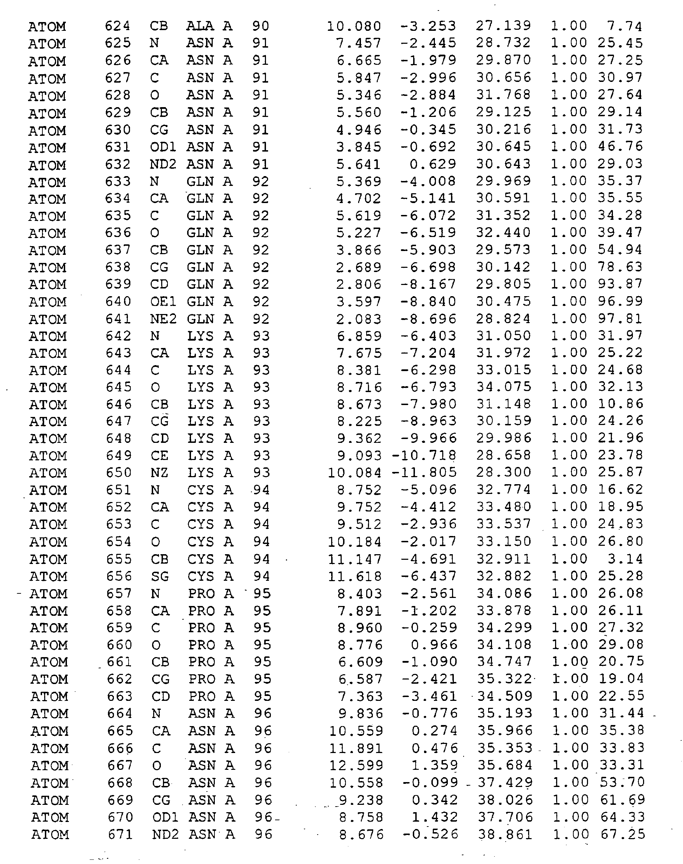

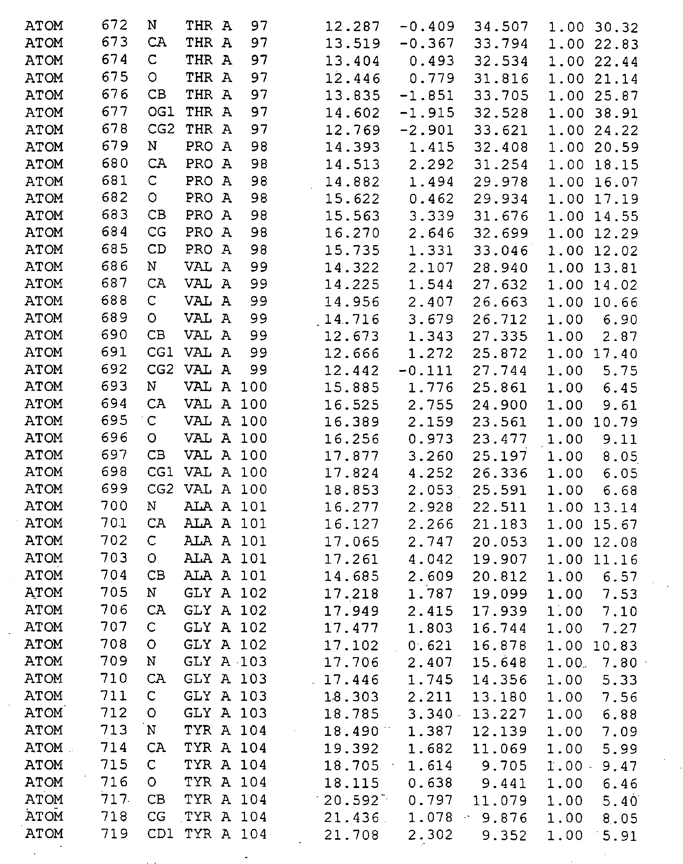

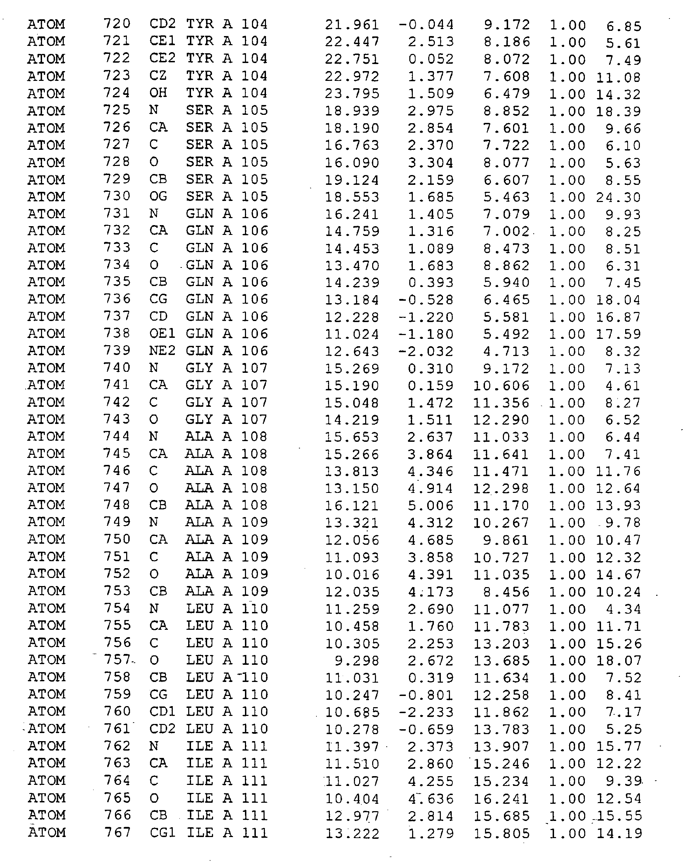

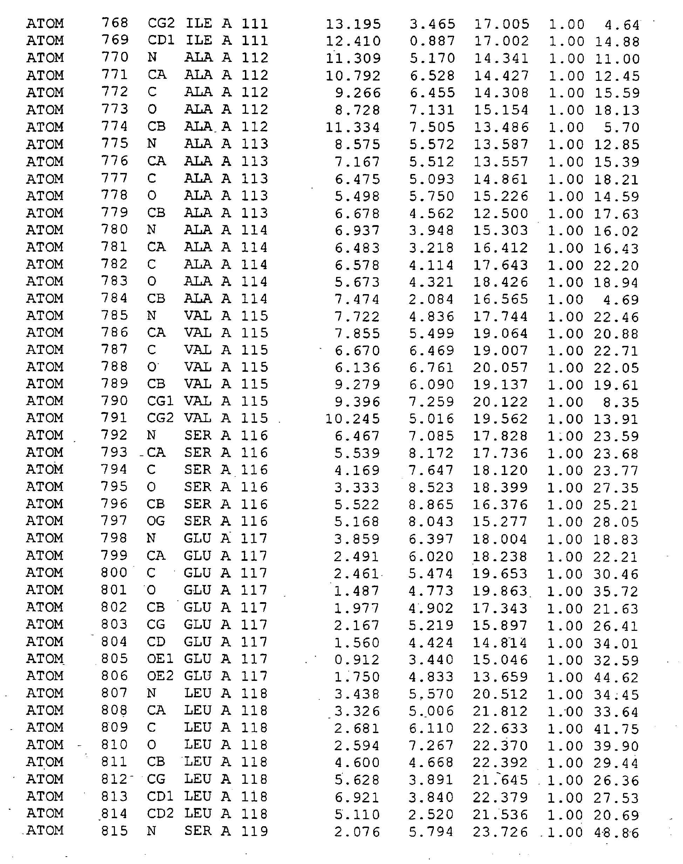

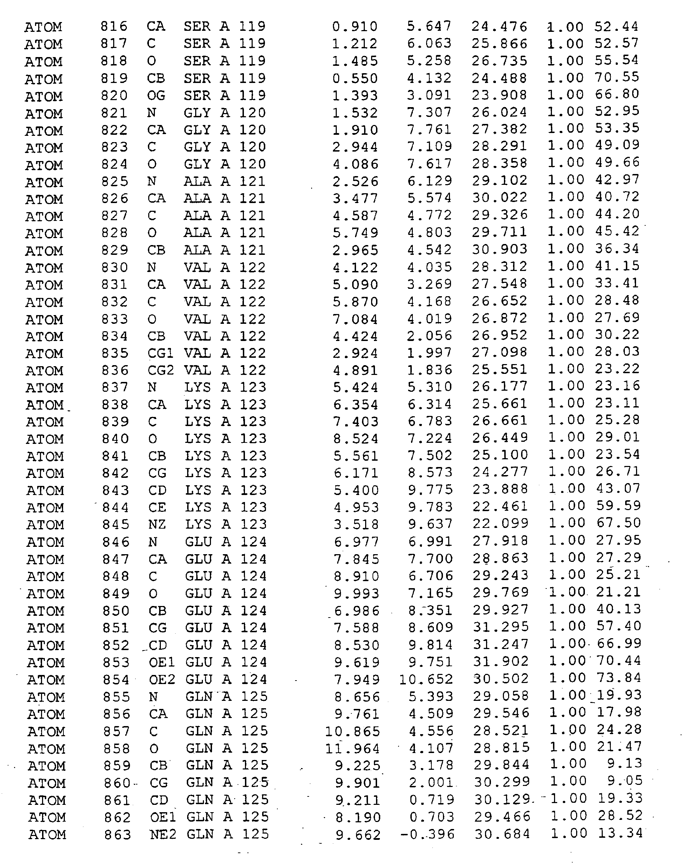

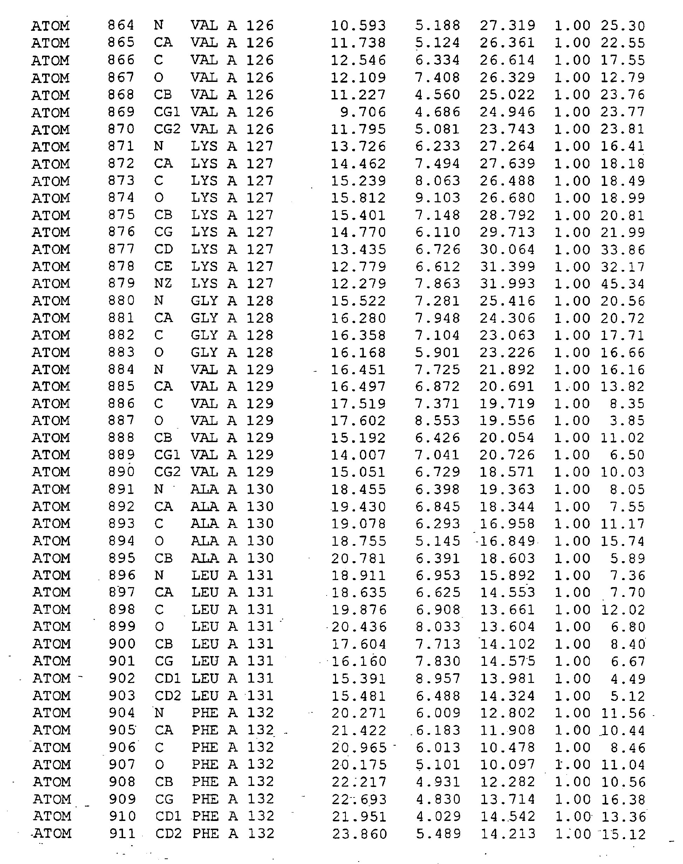

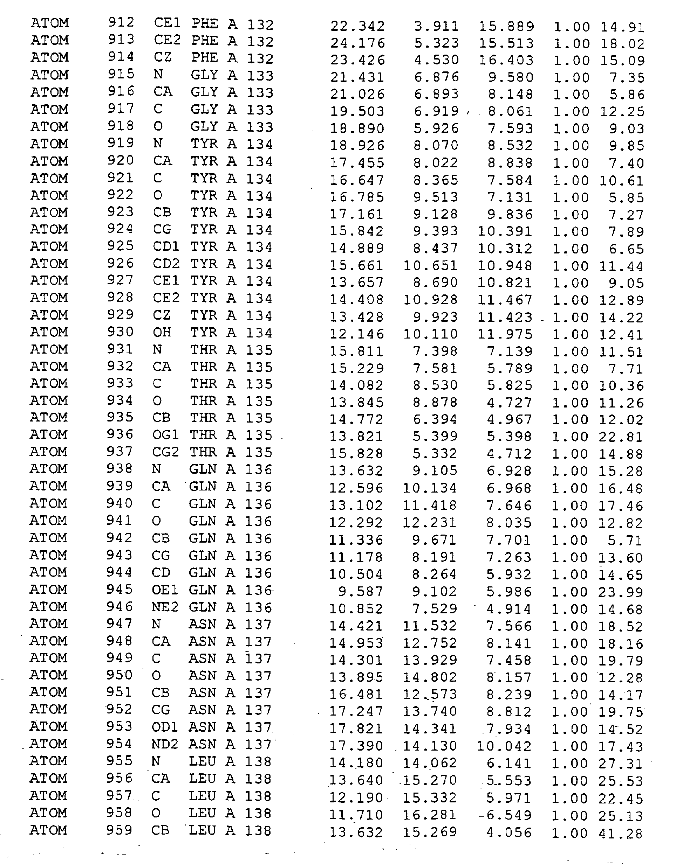

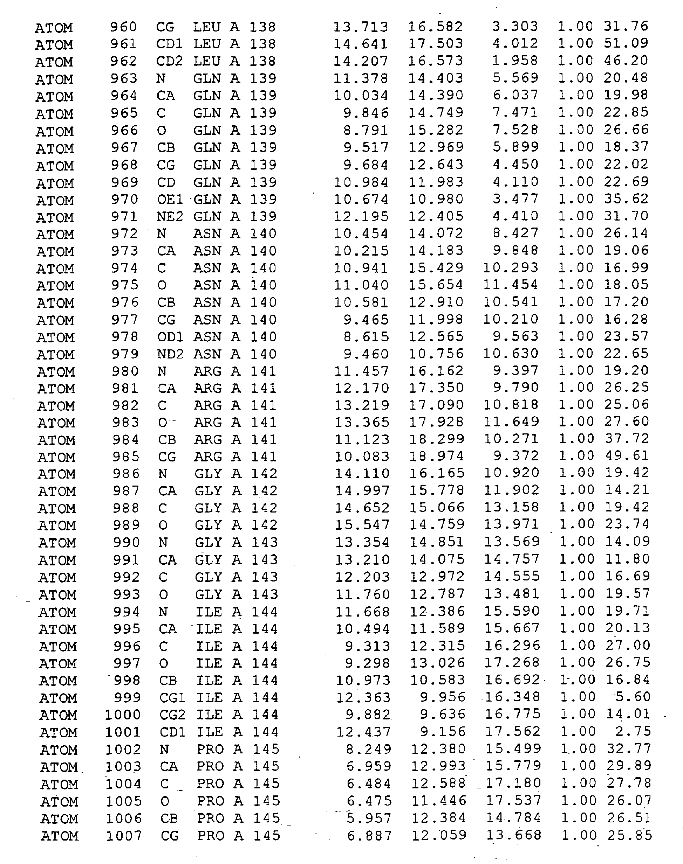

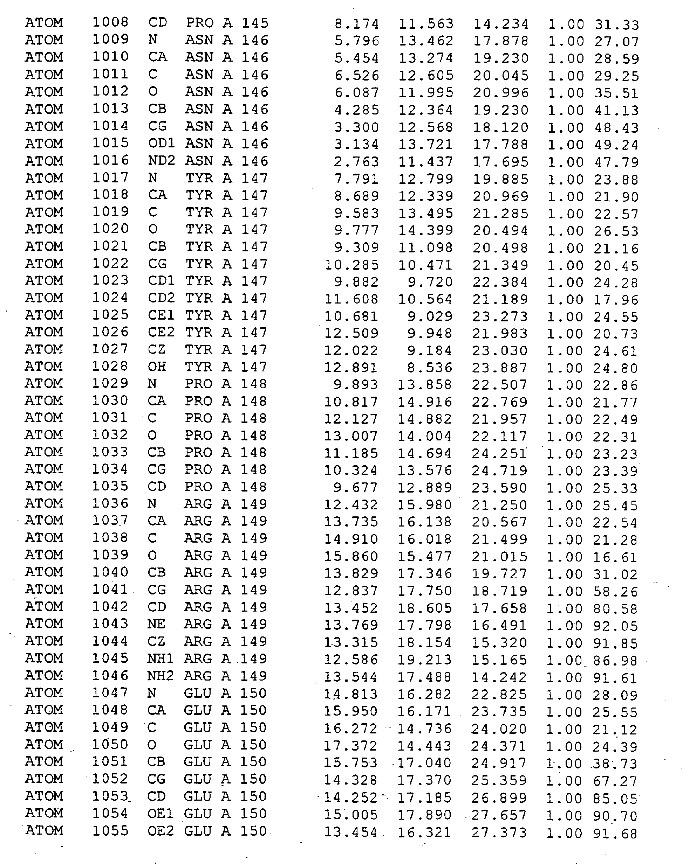

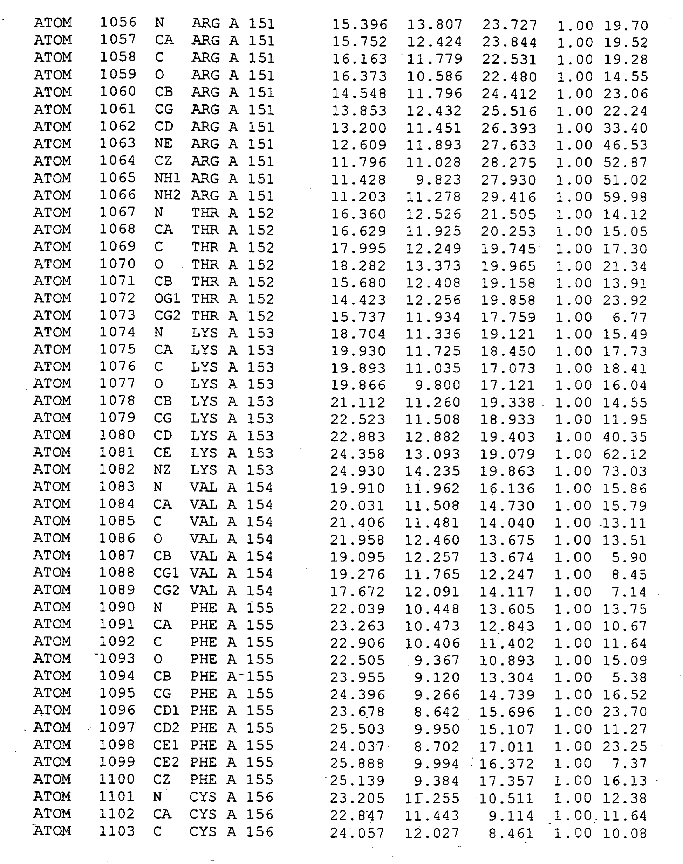

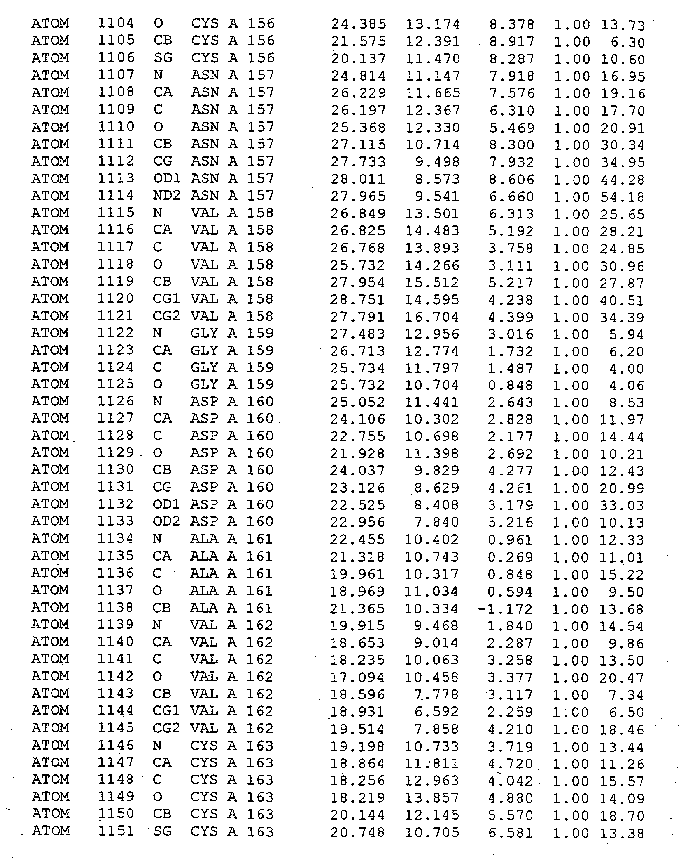

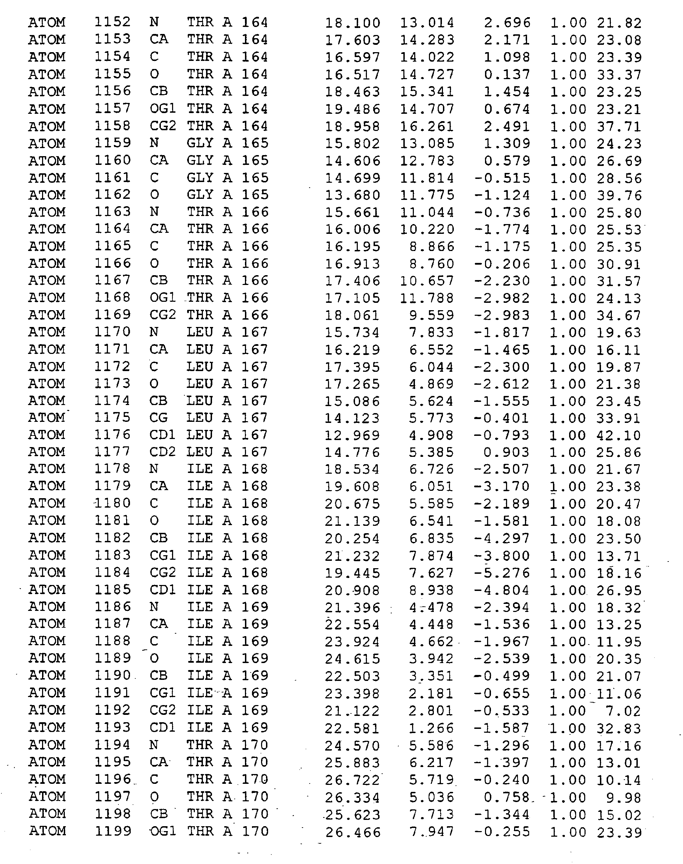

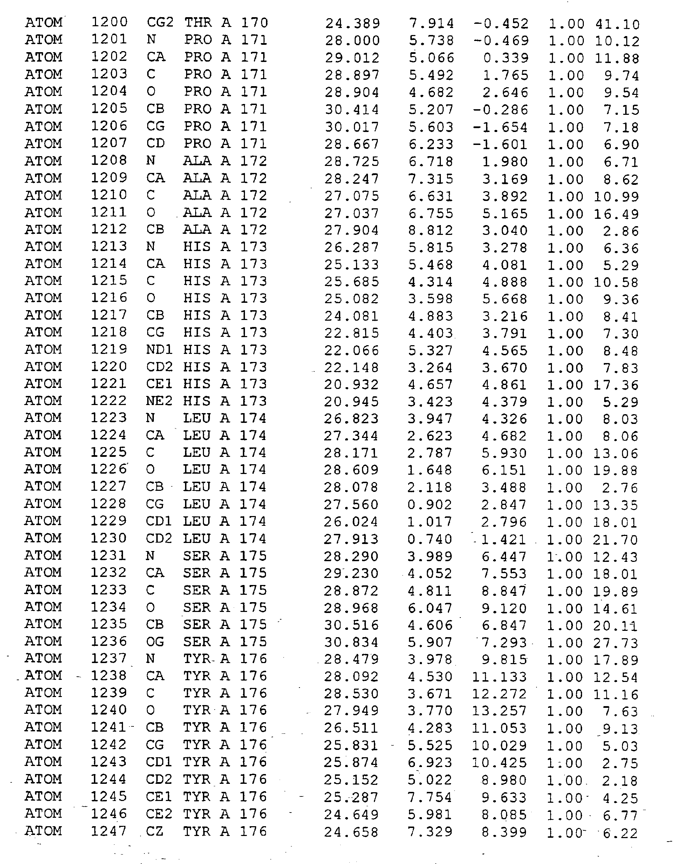

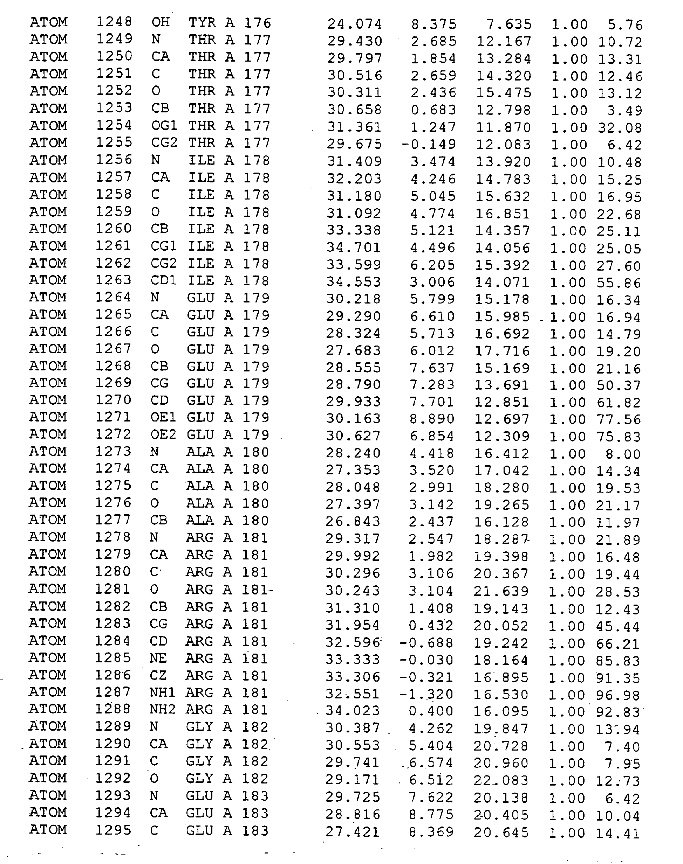

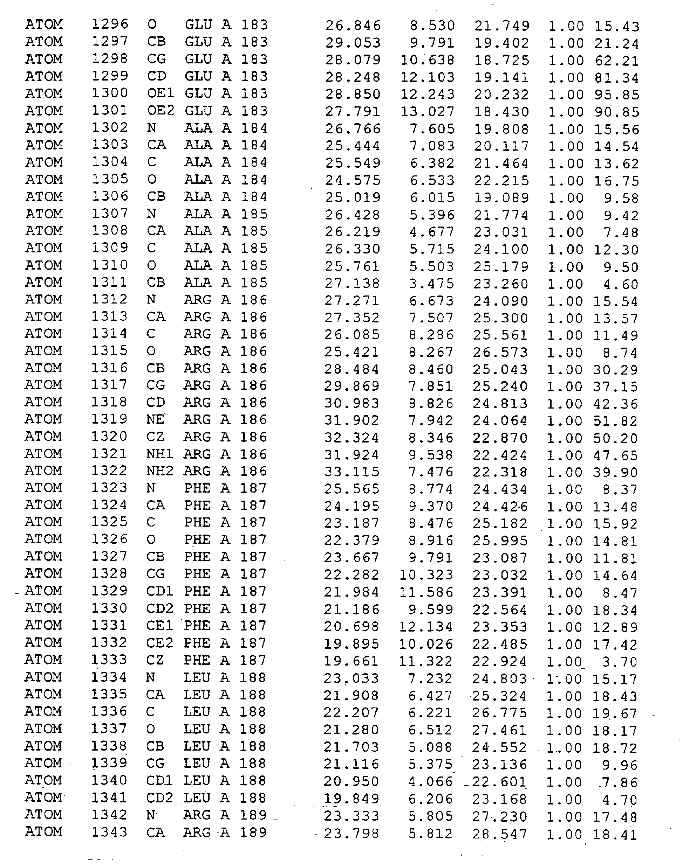

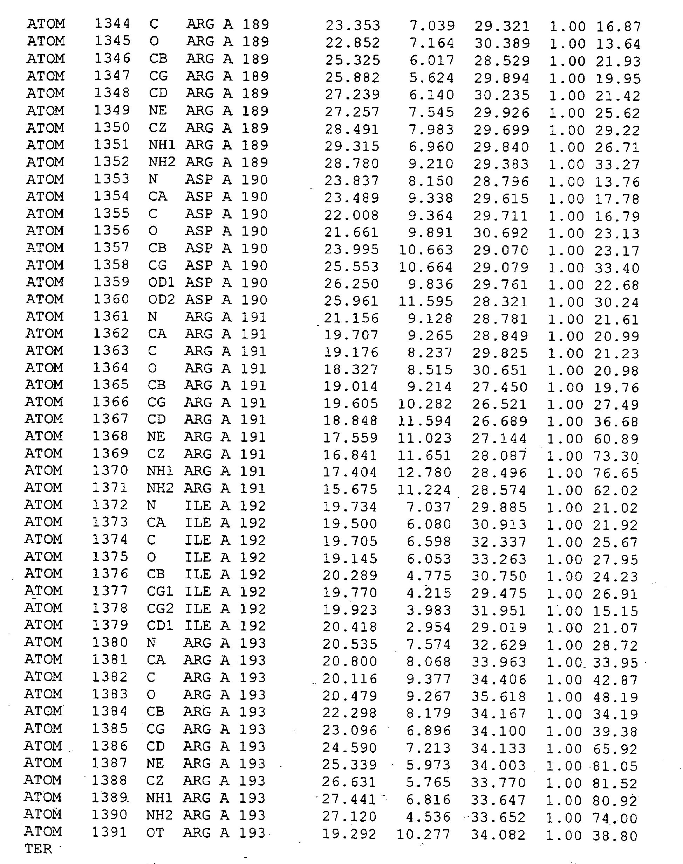

The amino acid sequence of the cutinase of *F. solani pisi* is shown as the mature peptide in Fig. 1 D of WO 94/14964. The numbering system used herein for the *F. solani pisi* cutinase is that used in WO 94/14964; it includes the pro-sequence shown in said Fig. 1 D; thus, the mature cutinase is at positions 16-214.

The parent cutinase may have an amino acid sequence which is at least 50 % (particularly at least 70 % or at least 80 %) homologous to the cutinase of *H. insolens* strain DSM 1800. The parent cutinase may particularly be one that can be aligned with the cutinase of *H. insolens* strain DSM 1800.

### Nomenclature for amino acids and alterations

The specification and claims refer to amino acids by their one-letter codes. A particular amino acid in a sequence is identified by its one-letter code and its position, e.g. Q1 indicates Gln (glutamine at position 1, i.e. at the N-terminal.

The nomenclature used herein for defining substitutions is basically as described in WO 92/05249. Thus, R51 P indicates substitution of R (Arg) at position 51 with P (Pro).

### Homology and alignment

For purposes of the present invention, the degree of homology may be suitably determined according to the method described in Needleman, S.B. and Wunsch, C. D., (1970), Journal of Molecular Biology, 48, 443-45, with the following settings for polypeptide sequence comparison: GAP creation penalty of 3.0 and GAP extension penalty of 0.1. The determination may be done by means of a computer program known such as GAP provided in the GCG program package (Program Manual for the Wisconsin Package, Version 8, August 1994, Genetics Computer Group, 575 Science Drive, Madison, Wisconsin, USA 53711).

Two given sequences can be aligned according to the method described in Needleman (*supra*) using the same parameters. This may be done by means of the GAP program (*supra*)*.*

### Three-dimensional structure of cutinase

The structure of the cutinase of *H. insolens* was solved in accordance with the principle for X-ray crystallographic methods as given, for example, in X-Ray Structure Determination, Stout, G.K. and Jensen, L.H., John Wiley & Sons, Inc. NY, 1989. The structural coordinates for the solved crystal structure at 2.2 Å resolution using the isomorphous replacement method are given in Fig. 1 in standard PDB format (Protein Data Bank, Brookhaven National Laboratory, Brookhaven, CT).

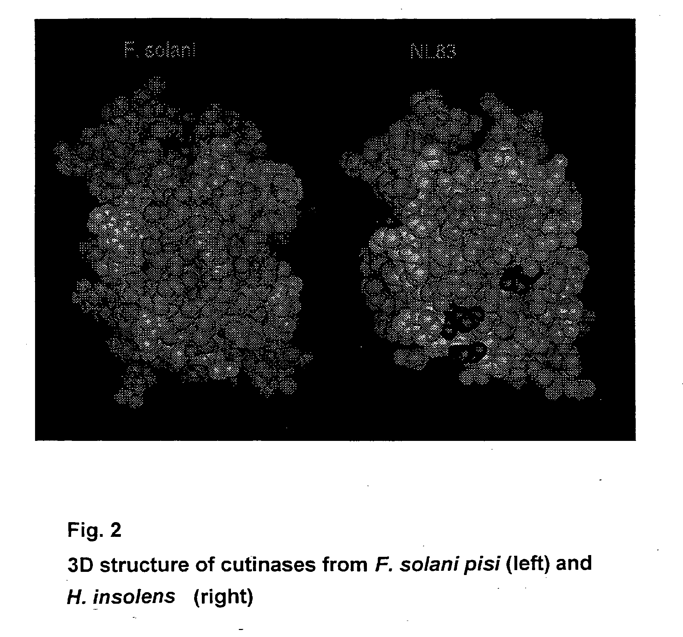

The structure of the cutinase of *F. solani pisi* is described in Martinez et al. (1992) Nature 356, 615-618. The 3D structures of the cutinases of *F. solani pisi* and *H. insolens* are compared as a computer model in Fig. 2.

It should be noted that the overall three-dimensional structures of fungal cutinases are very similar and have been shown by X-ray crystallography to be highly homologous. The similarities between the cutinases from *F. solani pisi* and *H. insolens* are clearly apparent from the computer model in Fig. 2. Therefore, modifications of the type indicated for one fungal cutinase will also be functional for other fungal cutinases.

### Substitution near N-terminal

The variant of the invention has one or more amino acid substitutions in the vicinity of the N-terminal. The substitution is within a distance of 17 Å (e.g. within 12 Å) and/or within 20 positions (e.g. within 15 positions) of the N-terminal. The distance from the N-terminal is to be calculated between the Cα atom of the amino acids, and is calculated from an amino acid in a crystal structure (i.e. visible in the X-ray structure).

In the cutinase of *H. insolens* strain DSM 1800, the two N-terminal amino acids (Q1 and L2. i.e. Gln and Leu at positions 1 and 2) are not visible in the X-ray structure, so the distance is to be calculated from amino acid G3. Amino acids within 17 Å include positions 3-12, 18, 20-60, 62-64, 82, 85-86, 100-108, 110-111, 130-132, 174, 176-182, 184-185, 188, and 192. Those within 12 Å include positions 3-8, 22-27, 30-47, 53-59, 102, 177, and 180-181.

In the cutinase of *F. solani pisi,* the N-terminal amino acid G17 is visible in the X-ray structure. Amino acids within 17 Å include positions 17-26, 34-75, 77-79, 101, 115, 117-119, 147, 191-197, 199-200, and 203. Those within 12 Å include positions 17-22, 38, 40, 45-58, 60, 65, and 70-72.

The variants of the invention have improved thermostability compared to the parent enzyme. The thermostability may be determined from the denaturation temperature by DSC (differential scanning calorimetry), e.g. as described in an example, e.g. at pH 8.5 with a scan rate of 90 K/hr. The variants may have a denaturation temperature which is at least 5°C higher than the parent enzyme.

The total number of substitutions in the above regions is typically 1-10, e.g. 1-5 substitutions in the above regions. In addition, the cutinase variant of the invention may optionally include other modifications of the parent enzyme, typically 10 or fewer, e.g. 5 or fewer alterations (substitutions, deletions or insertions) outside of the above regions. Thus, the total amino acid sequence of the variant typically 1-20, e.g. 1-10 alterations compared to the parent cutinase.

### Solvent accessible surface

One or more of the substitutions may be made at an exposed amino acid residue, i.e. an amino acid residue having a solvent accessible surface. This can be calculated by the "dssp" program (version October 1988) described in W. Kabsch and C. Sander, Biopolymers, 22 (1983) pp. 2577-2637.

In the cutinase of *H. insolens* strain DSM 1800, the following amino acids lie within 17 Å of G3 at the N-terminal and have a solvent accessible surface greater than 0: 3-12, 18, 26-33, 36-38, 40-45, 47-56, 59-60, 62-64, 82, 85-86, 104-105, 174, 176-179, 181-182, 192.

### Specific substitutions

The substitution near the N-terminal may specifically be one that increases the electrical charge, i.e. a substitution of a negatively charged amino acid with a neutral or positively charged amino acid or substitution of a neutral amino acid with a positively charged amino acid. Thus, a negative amino acid residue at a position corresponding to position E6, E10, E30, E47 D63, E82 and/or E179 in the cutinase of *Humicola insolens* strain DSM 1800 may be substituted by a neutral or positive amino acid, e.g. R, K, Y, H, Q or N. Some specific substitutions are those corresponding to E6Q/N, E10Q/N, E47K/R or E179Q/N. Also, a neutral amino acid residue at a position corresponding to N7, S11, N44 or N52 in the *H. insolens* cutinase may be substituted by a positive amino acid (R, K or H).

Another example of a substitution near the N-terminal is substitution with a Pro residue, e.g. a substitution corresponding to A14P or R51 P in the cutinase of *Humicola insolens* strain DSM 1800.

### Specific variants

The following are some examples of variants in the *H. insolens* cutinase. Corresponding variants may be made on the basis of other parent cutinases.
R51 P
E6N/Q+L1381
A14P+ E47K
E47K
E179N/Q
E6N/Q+E47K+R51P
A14P+ E47K+ E179N/Q
E47K+E179N/Q
E47K+ D63N
E6N/Q+ E10N/Q+ A14P+ E47K+ R51 P+ E179N/Q
E6N/Q+ A14P+ E47K+ R51 P+ E179N/Q
Q1 P+ L2V+ S11C+ N15T+ F24Y+ L46I+ E47K

### Use of cuti nase variant

The cutinase variant of the invention may be used, e.g., for the enzymatic hydrolysis of cyclic oligomers of poly(ethylene terephthalate), such as cyclic tri(ethylene terephthalate), abbreviated as c3ET.

In particular, this may be used to remove such cyclic oligomers from polyester containing fabric or yarn by treating the fabric or yarn with the cutinase variant, optionally followed by rinsing the fabric or yarn with an aqueous solution having a pH in the range of from about pH 7 to about pH 11. The treatment of polyester is conveniently carried out above the glass transition temperature of c3ET (about 55°C) and below the glass transition temperature of polyester (about 70°C). Thus, the treatment may suitably be carried out at 50-80°C, e.g. at 60-75°C. The process may be carried out in analogy with WO 97/27237.

The cutinase variant may be used to treat polyester-containing textile. e.g. PET (polymer of ethyleneglycol and terephthalic acid), P3GT (polymer of 1,3-propanediol and terephthalic acid) or a polyester/cotton blend. The treatment may provide benefits to the polyester textile such as improved wear and comfort, increased water permeability, reduced antistatic behavior, improve handle and softness, changed redeposition characteristics and/or color clarification.

The cutinase variant may be used to improve the functional finish of a PET-containing yarn or fabric by a treatment with the cutinase variant, followed by a treatment with a finishing agent such as a softener, an anti-crease resin, an anti-static agent, an anti-soiling agent or agents to impair wrinkle-free, permanent press ior fire resistance effects. The treatment with the cutinase variant may increase the number of functional groups in the surface, and this can be used to attach the functional finish. Examples of finishing agents are described in "SENSHOKU SIAGEKAKO BENRAN" published 1998-10-15 by Nihon Seni Sentaa KK.

The cutinase variant of the invention is also useful in detergents, where it may be incorporated to improve the removal of fatty soiling, as described in WO 94/03578 and WO 94/14964. The addition of the cutinase variant to laundry detergent may reduce malodor from cloth which is accumulated during several wash/wear-cycles.

The cutinase variant may also be used for degradation and recycling of polyester such as polycaprolactone (PCL), poly-ethyleneglycol-terephthalate (PET), polylactic acid, polybutylenesuccinate, and poly(hydroxybutiric acid)-co-(hydroxyvaleric acid), e.g. film and bottles, e.g. as described in JP-A 5-344897.

The cutinase variant may also be used for other known applications of lipases and cutinases, for example, in the baking industry (e.g. as described in WO 94/04035 and EP 585988), in the papermaking industry (e.g. for pitch removal, see EP 374700), and in the leather, wool and related industries (e.g. for degreasing of animal hides, sheepskin or wool), and for other applications involving degreasing/defatting. It may be used in immobilized form in the fat and oil industry, as a catalyst in organic synthesis (e.g. esterification, transesterification or ester hydrolysis reactions).

### Dyeing polyester

The invention provides a process for dyeing polyester fabric or yarn. In this process, the fabric or yarn is first treated with a cutinase, e.g. 12-48 hours at 50-70°C or 65-70°C, pH 7-10, followed by dyeing with dye, e.g. a reactive dye, a disperse dye or a cationic dye. The reactive dye may be one that reacts with OH or COOH groups, e.g. having the structure Chromophore-NHPh-SO₂CH₂CH₂OSO₃Na. The dyeing may be conducted at 40-80°C, e.g. for 20-60 minutes.

The cutinase may be a thermostable cutinase having a thermal denaturation temperature, T_{d}, at pH 8.5 which is at least 5° higher than the parent cutinase, e.g. 7-10° higher, e.g. a value of 65°C or higher. The measurement may be made by DSC as described in an Example of this specification.

### Surfactant

In the treatment of fabric or yarn, a conventional wetting agent and/or a dispersing agent may be used to improve the contact with the enzyme. The wetting agent may be a nonionic surfactant, e.g. an ethoxylated fatty alcohol. A very useful wetting agent is an ethoxylated and propoxylated fatty acid ester such as Berol 087 (product of Akzo Nobel, Sweden).

The dispersing agent may suitably be selected from nonionic, anionic, cationic, ampholytic or zwitterionic surfactants. More specifically, the dispersing agent may be selected from carboxymethylcellulose, hydroxypropylcellulose, alkyl aryl sulfonates, long-chain alcohol sulfates (primary and secondary alkyl sulfates), sulfonated olefins, sulfated monoglycerides, sulfated ethers, sulfosuccinates, sulfonated methyl ethers, alkane sulfonates, phosphate esters, alkyl isothionates, acylsarcosides, alkyltaurides, fluorosurfactants, fatty alcohol and alkylphenol condensates, fatty acid condensates, condensates of ethylene oxide with an amine, condensates of ethylene oxide with an amide, sucrose esters, sorbitan esters, alkyloamides, fatty amine oxides, ethoxylated monoamines, ethoxylated diamines, alcohol ethoxylate and mixtures thereof. A very useful dispersing agent is an alcohol ethoxylate such as Berol 08 (product of Akzo Nobel, Sweden).

### Methods for preparing cutinase variants

The cutinase variant of the invention can be prepared by methods known in the art, e.g. as described in WO 94/14963 or WO 94/14964 (Unilever). The following describes methods for the cloning of cutinase-encoding DNA sequences, followed by methods for generating mutations at specific sites within the cutinase-encoding sequence.

### Cloning a DNA sequence encoding a cutinase

The DNA sequence encoding a parent cutinase may be isolated from any cell or microorganism producing the cutinase in question, using various methods well known in the art. First, a genomic DNA and/or cDNA library should be constructed using chromosomal DNA or messenger RNA from the organism that produces the cutinase to be studied. Then, if the amino acid sequence of the cutinase is known, labeled oligonucleotide probes may be synthesized and used to identify cutinase-encoding clones from a genomic library prepared from the organism in question. Alternatively, a labeled oligonucleotide probe containing sequences homologous to another known cutinase gene could be used as a probe to identify cutinase-encoding clones, using hybridization and washing conditions of lower stringency.

Yet another method for identifying cutinase-encoding clones would involve inserting fragments of genomic DNA into an expression vector, such as a plasmid, transforming cutinase-negative bacteria with the resulting genomic DNA library, and then plating the transformed bacteria onto agar containing a substrate for cutinase (i.e. maltose), thereby allowing clones expressing the cutinase to be identified.

Alternatively, the DNA sequence encoding the enzyme may be prepared synthetically by established standard methods, *e.g*. the phosphoroamidite method described S.L. Beaucage and M.H. Caruthers, (1981), Tetrahedron Letters 22, p. 1859-1869, or the method described by Matthes et al., (1984), EMBO J. 3, p. 801-805. In the phosphoroamidite method, oligonucleotides are synthesized, *e.g*. in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al., (1988), Science 239, 1988, pp. 487-491.

### Site-directed mutagenesis

Once a cutinase-encoding DNA sequence has been isolated, and desirable sites for mutation identified, mutations may be introduced using synthetic oligonucleotides. These oligonucleotides contain nucleotide sequences flanking the desired mutation sites. In a specific method, a single-stranded gap of DNA, the cutinase-encoding sequence, is created in a vector carrying the cutinase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al., (1984), Biotechnology 2, p. 646-639. US 4,760,025 discloses the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method for introducing mutations into cutinase-encoding DNA sequences is described in Nelson and Long, (1989), Analytical Biochemistry 180, p. 147-151. It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

### Expression of cutinase variants

According to the invention, a DNA sequence encoding the variant produced by methods described above, or by any alternative methods known in the art, can be expressed, in enzyme form, using an expression vector which typically includes control sequences encoding a promoter, operator, ribosome binding site, translation initiation signal, and, optionally, a repressor gene or various activator genes.

### Expression vector

The recombinant expression vector carrying the DNA sequence encoding a cutinase variant of the invention may be any vector which may conveniently be subjected to recombinant DNA procedures, and the choice of vector will often depend on the host cell into which it is to be introduced. The vector may be one which, when introduced into a host cell, is integrated into the host cell genome and replicated together with the chromosome(s) into which it has been integrated. Examples of suitable expression vectors include pMT838.

### Promoter

In the vector, the DNA sequence should be operably connected to a suitable promoter sequence. The promoter may be any DNA sequence which shows transcriptional activity in the host cell of choice and may be derived from genes encoding proteins either homologous or heterologous to the host cell.

Examples of suitable promoters for directing the transcription of the DNA sequence encoding a cutinase variant of the invention, especially in a bacterial host, are the promoter of the *lac* operon of *E.coli,* the *Streptomyces coelicolor* agarase gene *dag*A promoters, the promoters of the *Bacillus licheniformis* α-amylase gene (*amyL*)*,* the promoters of the *Bacillus stearothermophilus* maltogenic amylase gene (*amyM*)*,* the promoters of the *Bacillus amyloliquefaciens* α-amylase (*amyQ*)*,* the promoters of the *Bacillus subtilis* xylA and xylB genes etc. For transcription in a fungal host, examples of useful promoters are those derived from the gene encoding *A*. *oryzae* TAKA amylase, the TPI (triose phosphate isomerase) promoter from *S*. *cerevisiae* (Alber et al. (1982), J. Mol. Appl. Genet 1, p. 419-434, *Rhizomucor miehei* aspartic proteinase, *A. niger* neutral α-amylase, *A. niger* acid stable α-amylase, *A. niger* glucoamylase, *Rhizomucor miehei* lipase, *A. oryzae* alkaline protease, *A. oryzae* triose phosphate isomerase or *A. nidulans* acetamidase.

### Expression vector

The expression vector of the invention may also comprise a suitable transcription terminator and, in eukaryotes, polyadenylation sequences operably connected to the DNA sequence encoding the α-amylase variant of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the *dal* genes from *B. subtilis* or *B. licheniformis,* or one which confers antibiotic resistance such as ampicillin, kanamycin, chloramphenicol or tetracyclin resistance. Furthermore, the vector may comprise *Aspergillus* selection markers such as amdS, argB, niaD and sC, a marker giving rise to hygromycin resistance, or the selection may be accomplished by co-transformation, *e.g.* as described in WO 91/17243.

The procedures used to ligate the DNA construct of the invention encoding a cutinase variant, the promoter, terminator and other elements, respectively, and to insert them into suitable vectors containing the information necessary for replication, are well known to persons skilled in the art (cf., for instance, Sambrook et al., Molecular Cloning: A Laboratory Manual, 2nd Ed., Cold Spring Harbor, 1989).

### Host Cells

The cell of the invention, either comprising a DNA construct or an expression vector of the invention as defined above, is advantageously used as a host cell in the recombinant production of a cutinase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, e.g. by homologous or heterologous recombination. Alternatively, the cell may be transformed with an expression vector as described above in connection with the different types of host cells.

The cell of the invention may be a cell of a higher organism such as a mammal or an insect, but is preferably a microbial cell, e.g. a bacterial or a fungal (including yeast) cell.

Examples of suitable bacteria are Gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis,* or *Streptomyces lividans* or *Streptomyces murinus,* or gramnegative bacteria such as *E.coli.* The transformation of the bacteria may, for instance, be effected by protoplast transformation or by using competent cells in a manner known *per se.*

The yeast organism may favorably be selected from a species of *Saccharomyces* or *Schizosaccharomyces, e.g. Saccharomyces cerevisiae.*

The host cell may also be a filamentous fungus *e.g.* a strain belonging to a species of *Aspergillus,* most preferably *Aspergillus oryzae* or *Aspergillus niger,* or a strain of *Fusarium,* such as a strain of *Fusarium oxysporium, Fusarium graminearum* (in the perfect state named *Gribberella zeae,* previously *Sphaeria zeae, synonym with Gibberella roseum and Gibberella roseum* f. sp. *cerealis),* or *Fusarium sulphureum* (in the prefect state named *Gibberella puricaris,* synonym with *Fusarium trichothecioides, Fusarium bactridioides, Fusarium sambucium, Fusarium roseum,* and *Fusarium roseum var. graminearum), Fusarium cerealis* (synonym with *Fusarium crokkwellnse*)*,* or *Fusarium venenatum.*

In a preferred embodiment of the invention the host cell is a protease deficient or protease minus strain.

This may for instance be the protease deficient strain *Aspergillus oryzae* JaL 125 having the alkaline protease gene named "alp" deleted. This strain is described in WO 97/35956 (Novo Nordisk).

Filamentous fungi cells may be transformed by a process involving protoplast formation and transformation of the protoplasts followed by regeneration of the cell wall in a manner known per se. The use of *Aspergillus* as a host micro-organism is described in EP 238 023 (Novo Nordisk A/S), the contents of which are hereby incorporated by reference.

### Production of cutinase variant by cultivation of transformant

The invention relates, *inter alia,* to a method of producing a cutinase variant of the invention, which method comprises cultivating a host cell under conditions conducive to the production of the variant and recovering the variant from the cells and/or culture medium.

The medium used to cultivate the cells may be any conventional medium suitable for growing the host cell in question and obtaining expression of the cutinase variant of the invention. Suitable media are available from commercial suppliers or may be prepared according to published recipes (*e.g.* as described in catalogues of the American Type Culture Collection).

The cutinase variant secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### Expression of variant in plants

The present invention also relates to a transgenic plant, plant part or plant cell which has been transformed with a DNA sequence encoding the variant of the invention so as to express and produce this enzyme in recoverable quantities. The enzyme may be recovered from the plant or plant part. Alternatively, the plant or plant part containing the recombinant enzyme may be used as such.

The transgenic plant can be dicotyledonous or monocotyledonous, for short a dicot or a monocot. Examples of monocot plants are grasses, such as meadow grass (blue grass, Poa), forage grass such as festuca, lolium, temperate grass, such as Agrostis, and cereals, e.g. wheat, oats, rye, barley, rice, sorghum and maize (corn).

Examples of dicot plants are tobacco, legumes, such as lupins, potato, sugar beet, pea, bean and soybean, and cruciferous (family Brassicaceae), such as cauliflower, oil seed rape and the closely related model organism Arabidopsis thaliana.

Examples of plant parts are stem, callus, leaves, root, fruits, seeds, and tubers. In the present context, also specific plant tissues, such as chloroplast, apoplast, mitochondria, vacuole, peroxisomes and cytoplasm are considered to be a plant part. Furthermore, any plant cell, whatever the tissue origin, is considered to be a plant part.

Also included within the scope of the invention are the progeny of such plants, plant parts and plant cells.

The transgenic plant or plant cell expressing the variant of the invention may be constructed in accordance with methods known in the art. In short the plant or plant cell is constructed by incorporating one or more expression constructs encoding the enzyme of the invention into the plant host genome and propagating the resulting modified plant or plant cell into a transgenic plant or plant cell.

Conveniently, the expression construct is a DNA construct which comprises a gene encoding the enzyme of the invention in operable association with appropriate regulatory sequences required for expression of the gene in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences is determined, eg on the basis of when, where and how the enzyme is desired to be expressed. For instance, the expression of the gene encoding the enzyme of the invention may be constitutive or inducible, or may be developmental, stage or tissue specific, and the gene product may be targeted to a specific tissue or plant part such as seeds or leaves. Regulatory sequences are eg described by Tague et al, Plant, Phys., 86, 506, 1988.

For constitutive expression the 35S-CaMV promoter may be used (Franck et al., 1980. Cell 21: 285-294). Organ-specific promoters may eg be a promoter from storage sink tissues such as seeds, potato tubers, and fruits (Edwards & Coruzzi, 1990. Annu. Rev. Genet. 24: 275-303), or from metabolic sink tissues such as meristems (lto et al., 1994. Plant Mol. Biol. 24: 863-878), a seed specific promoter such as the glutelin, prolamin, globulin or albumin promoter from rice (Wu et al., Plant and Cell Physiology Vol. 39, No. 8 pp. 885-889 (1998)), a *Vicia faba* promoter from the legumin B4 and the unknown seed protein gene from *Vicia faba* described by Conrad U. et al, Journal of Plant Physiology Vol. 152, No. 6 pp. 708-711 (1998), a promotter from a seed oil body protein (Chen et al., Plant and cell physiology vol. 39, No. 9 pp. 935-941 (1998), the storage protein napA promoter from Brassica napus, or any other seed specific promoter known in the art, eg as described in WO 91/14772. Furthermore, the promoter may be a leaf specific promoter such as the rbcs promoter from rice or tomato (Kyozuka et al., Plant Physiology Vol. 102, No. 3 pp. 991-1000 (1993), the chlorella virus adenine methyltransferase gene promoter (Mitra, A. and Higgins, DW, Plant Molecular Biology Vol. 26, No. 1 pp. 85-93 (1994), or the aldP gene promoter from rice (Kagaya et al., Molecular and General Genetics Vol. 248, No. 6 pp. 668-674 (1995), or a wound inducible promoter such as the potato pin2 promoter (Xu et al, Plant Molecular Biology Vol. 22, No. 4 pp. 573-588 (1993).

A promoter enhancer element may be used to achieve higher expression of the enzyme in the plant. For instance, the promoter enhancer element may be an intron which is placed between the promoter and the nucleotide sequence encoding the enzyme. For instance, Xu et al. *op cit* disclose the use of the first intron of the rice actin 1 gene to enhance expression.

The selectable marker gene and any other parts of the expression construct may be chosen from those available in the art.

The DNA construct is incorporated into the plant genome according to conventional techniques known in the art, including *Agrobacterium-*mediated transformation, virus-mediated transformation, micro injection, particle bombardment, biolistic transformation, and electroporation (Gasser et al, Science, 244, 1293; Potrykus, Bio/Techn. 8, 535, 1990; Shimamoto et al, Nature, 338, 274, 1989).

Presently, *Agrobacterium tumefaciens* mediated gene transfer is the method of choice for generating transgenic dicots (for review Hooykas & Schilperoort, 1992. Plant Mol. Biol. 19: 15-38), however it can also be used for transforming monocots, although other transformation methods are generally preferred for these plants. Presently, the method of choice for generating transgenic monocots is particle bombardment (microscopic gold or tungsten particles coated with the transforming DNA) of embryonic calli or developing embryos (Christou, 1992. Plant J. 2: 275-281; Shimamoto, 1994. Curr. Opin. Biotechnol. 5: 158-162; Vasil et al., 1992. Bio/Technology 10: 667-674). An alternative method for transformation of monocots is based on protoplast transformation as described by Omirulleh S, et al., Plant Molecular biology Vol. 21, No. 3 pp. 415-428 (1993).

Following transformation, the transformants having incorporated the expression construct are selected and regenerated into whole plants according to methods well-known in the art.

### MATERIALS AND METHODS

### Plasmids

### pJSO026

This is a *S*. *cerevisiae* expression plasmid described in WO 97/07205 and in J.S.Okkels, (1996) "A URA3-promoter deletion in a pYES vector increases the expression level of a fungal lipase in Saccharomyces cerevisiae. Recombinant DNA Biotechnology III: The Integration of Biological and Engineering Sciences, vol. 782 of the Annals of the New York Academy of Sciences).

### pFuku83

This is a yeast and E. coli shuttle vector for expression of the H. insolens cutinase under the control of a TPI promoter, constructed from pJSO026.

### Su bstrate

### BETEB

Terephthalic acid bis(2-hydroxyethyl)ester dibenzoate is herein abbreviated as BETEB (benzoyl-ethylene-terephthalic-ethelene-benzoate). It was prepared from terephthalic acid bis (2-hydroxyethyl) ester and benzoic acid.

### Lipase activity (LU)

A substrate for lipase is prepared by emulsifying tributyrin (glycerin tributyrate) using gum Arabic as emulsifier. The hydrolysis of tributyrin at 30 °C at pH 7 is followed in a pH-stat titration experiment. One unit of lipase activity (1 LU) equals the amount of enzyme capable of releasing 1 µmol butyric acid/min at the standard conditions.

### Differential scanning calorimetry (DSC)

Sample and reference solutions are carefully degassed immediately prior to loading of samples into the calorimeter (reference: buffer without enzyme). Sample and reference solutions (approx. 0.5 ml) are thermally pre-equillibrated for 20 minutes at 5°C. The DSC scan is performed from 5 C to 95 C at a scan rate of approx. 90 K/hr. Denaturation temperatures are determined at an accuracy of approx. +/- 1 C. A VP-DSC from MicroCal Inc. is suitable for the experiments.

### Methods

### PCR conditions

step 1: 94° C, 120 sec.
step 2: 94° C, 60 sec
step 3: 50° C, 60 sec
step 4: 72° C, 150 sec.
Go to step 2, 35 cycles
step 5: 72° C, 480 sec.
Step 6: 4° C, for ever

### EXAMPLES

### Example 1: Preparation of cutinase variants

A DNA sequence encoding *H. insolens* cutinase was obtained as described in US 5,827,719 (Novo Nordisk) and was found to have the DNA sequence shown in SEQ ID NO: 1 therein.

Variants were prepared by localized random mutagenesis and selection of positive clones by incubation at 60°C for 1 day on BETEB plates. The BETEB plates contained 200 ml/I of 500 mM glycine buffer (pH 8.5), 1.25 g/l of BETEB (dissolved in hot ethanol) and 20 g/l of agar.

Three positive variants were isolated, and their amino acid sequence was determined. They were found to have the following modifications, compared to the parent *H. insolens* cutinase:
A14P + E47K
E47K
E179Q

### Example 2: Site directed mutation

A variant of the *H*. insolens cutinase having the substitutions E6Q+ E47K+ R51 P was prepared as follows:

A pair of PCR primers were designed so as to introduce amino acid substitutions, making use of the existed restriction enzyme sites nearby, as follows (an asterisk indicates an introduced mutation):
Upper primer: E6Q F
   egg cag ctg gga gcc atc c*ag aac
*Pvu* II
Lower primer: E47K,R51 P
   cgc cct gga tcc aga tgt tcg* gga tgt ggg act t*aa ggc
*Bam*H I

PCR was run using these primers and pFukuNL83 as a template under the PCR condition described above.

The obtained PCR fragment was purified by Clontech Spincolumn and digested with *Pvu* II and *Bam*H *I.*

The resultant fragment was gel-purified and ligated to pFukuNL83 which had been digested with the same restriction enzyme sites.

### Example 3: Thermostability of cutinase variants

### Variants

The thermostability was tested as described below for the *H. insolens* cutinase and the following variants thereof:
A14P+ E47K
E47K
E179Q
E6Q+ E47K+ R51P
A14P+ E47K+ E179Q
E6Q+ A14P+ E47K+ R51 P+ E179Q
E6Q+ E1 OQ+ A14P+ E47K+ R51P+ E179Q

### Differential Scanning Calorimetry (DSC)

Thermostability of cutinase variants was investigated by means of DSC at pH 4.5 (50 mM acetate buffer) and pH 8.5 (50mM glycyl-glycine buffer). The thermal denaturation temperature, T_{d}, was taken as the top of denaturation peak (major endothermic peak) in thermograms (Cp vs. T) obtained after heating of enzyme solutions at a constant programmed heating rate.

The parent cutinase was found to have T_{d} of 63°C at pH 8.5. Six of the above variants were found to have T_{d} of 70-73°C, i.e. an improvement of 7-10°.

The parent cutinase was found to have T_{d} of 61 °C at pH 4.5. Five of the above variants were found to have T_{d} of 64-66°C, i.e. an improvement of 3-5°.

### Hydrolysis of BETEB

The thermostability of the *H. insolens* cutinase and two of the above variants was measured by hydrolysis of BETEB at elevated temperature. For each cutinase, the following mixture was incubated for 17 hours at various temperatures in the range 55-70°C:
0.1 ml 0.5 M glycyl-glycine buffer (pH 8.5)
0.1 ml 0.5 % BETEB dissolved in ethanol
0.1 ml enzyme solution (approx. 25 LU/ml)
0.7 ml Milli Q water

The degree of hydrolysis was measured after the incubation. The results are shown in the table below.

| | Variant | Variant | Parent |
|---|---|---|---|
| | 27 LU/ml | 25 LU/ml | 24 LU/ml |
| 55°C | 98 % | 99 % | 72 % |
| 60°C | 91 % | 83 % | 33 % |
| 65°C | 66 % | 13 % | 7 % |

These results clearly show that the variants have improved thermostability compared to the parent cutinase.

### Hydrolysis of BETEB

The thermostability of the *H. insolens* cutinase and three of the above variants was measured by hydrolysis of BETEB at 60°C for 2 hours. The hydrolysis was carried out at the above conditions, except that the temperature was fixed at 60°C and the cutinase dosage was varied. The results below are shown in the table below.

| LU/ml | Variant | Variant | Variant | Parent |
|---|---|---|---|---|
| 0 | 0% | 0% | 0% | 0% |
| 10 | 97% | 99% | 9% | 6% |
| 20 | 98 % | 99 % | 74 % | |
| 50 | 98% | 94% | 93% | 15% |
| 100 | 88 % | 69 % | 92 % | 34 % |
| 300 | | | | 41 % |
| 600 | | | | 63 % |
| 1200 | | | | 82 % |

The results show a much faster hydrolysis at 60°C with the variants than with the parent cutinase.

### Example 4: Hydrolysis of c3ET

The *H. insolens* cutinase and five of the above variants were tested in hydrolysis of c3ET at elevated temperature. For each cutinase, the following mixture was incubated for 2 hours at various temperatures.

0.115mg c3ET (0.1 ml of 2mM c3ET dissolved in HFIP was taken in reaction vessel. Solvent was removed under vacuum, then dried up at 70°C over night)

| | |
|---|---|
| 0.1 ml | 0.5M glycyl-glycine buffer (pH8.5) |
| 0.1 ml | enzyme solution (approx. 600LU/ml) |
| 0.8ml | Milli Q water |

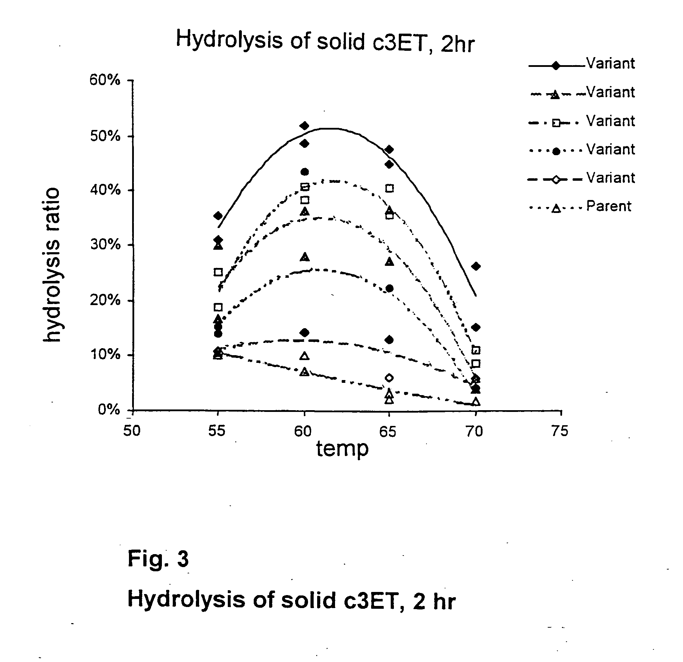

After the incubation, 2ml of 1,1,1,3,3,3-Hexafluoro-2-propanol (HFIP) was added to each reaction mixture, then hydrolysis ratio was measured by HPLC. The results shown in Fig 3 clearly indicate that the variants have improved thermostability compared to the parent cutinase.

### Example 5: Hydrolysis of c3ET on yarn

The thermostability of the *H. insolens* cutinase five of the above variants was tested using polyester yarn containing c3ET as by product. The following substrate mixture was preincubated at 60 or 65°C:

| | |
|---|---|
| 0.1 g | polyester yarn |
| 0.2ml | 0.5M glycyl-glycine buffer (pH8.5) |
| 1.7ml | Milli Q water |

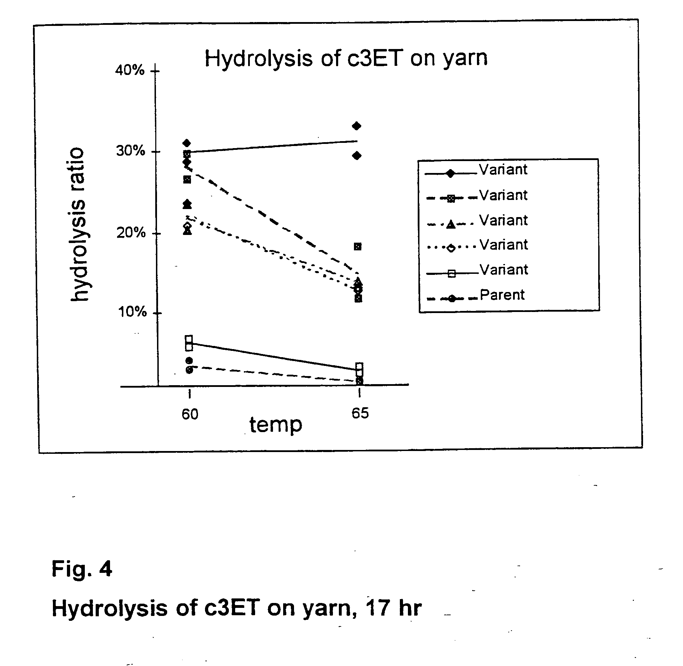

After preincubation, 0.1ml enzyme solution (approx. 1000 LU/ml) was added to each reaction vessel and incubated for 17 hours. Then 2ml HFIP was added and left for 30 minutes to extract and hydrolyze c3ET sitting on the surface of the polyester yarn; then the hydrolysis ratio was measured. The results are shown in Fig. 4.

It is seen that the variants are more effective than the parent cutinase for hydrolyzing c3ET on polyester yarn. One variant gives higher hydrolysis ratio at 65°C than at 60°C.

### Example 6: Treatment of yarn with cutinase variant

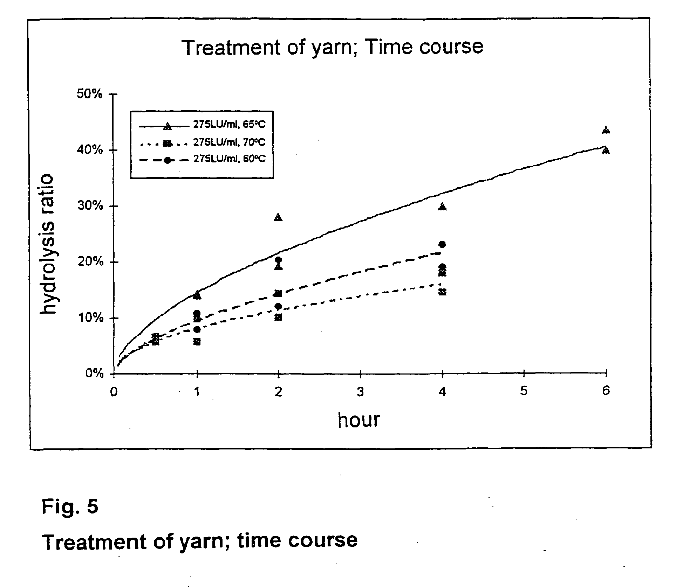

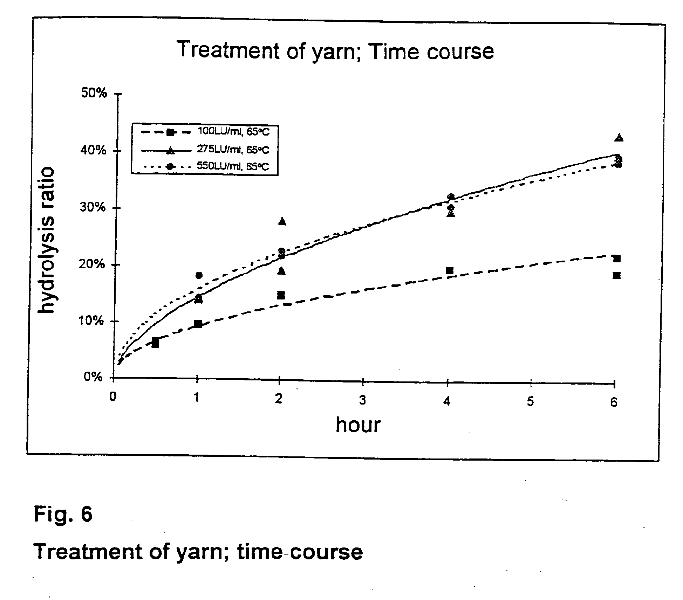

Time courses of c3ET hydrolysis on polyester yarn at different temperature or dosage were examined. Time course at different temperatures is shown in Fig 5. It is seen that the optimum temperature is 65°C. At 70°C there is still about half of the activity left. Time course with increased enzyme dosage is shown in Fig 6. The curves at dosage 275 and 550 LU/ml are seen to be the same, indicating that the hydrolysis ratio reached to plateau between dosage of 100 to 275 LU/ml. Presumably 200LU/ml is enough.

### Example 7: Dyeing polyester with reactive dye

The following polyester fabrics were treated:
woven fabric; ca. 2 x 2 cm, 34mg
knitted fabric; ca. 1.5 x 1.5 cm, 50mg

Each fabric was soaked in 0.9 ml, 50 mM GlyGly (glycyl-glycine) buffer (pH 8.5) and 0.1 ml solution of a variant of the *H. insolens* cutinase (1100 LU/ml), and incubated at 65 or 70°C. After one day, another 0.1 ml enzyme solution was added, incubation was continued for two more days, the fabrics were then taken out and rinsed in water. A comparative experiment was made with the parent cutinase, and a blank was treated in the same man- ner without enzyme.

The fabrics were stirred in a mixture of 9 g 120 g Na₂SO₄ and 60 g Na₂CO₃ in 3 liter deionized water at 60 °C for 30 min, and then rinsed with running warm water. The reactive dye was Celmazol Brilliant Blue B (product of Mitsui Chemical Co., Japan), which has the structure Chromophore-NHPh-SO₂CH₂CH₂OSO₃Na.

In all four experiments, (woven and knitted, 65 and 70°C), the fabrics were uniformly dyed.

### Example 8: Solubilization of polyester fragments from knitted textile

A 1 x1 cm sample of knitted polyester textile (PET, polymer of ethyleneglycol and terephthalic acid) was incubated for 1 hour in 1 ml of buffer at pH 10, 60°C with 0.01 mg of a variant of *H. insolens* cutinase. The reaction mixture was separated, and the release of terephthalic acid was found by measuring OD at 250 nm (expressed as OD₂₅₀/mg PET). comparative experiments were made without enzyme or with the parent cutinase. Results:

| | Enzyme | OD₂₅₀ |
|---|---|---|
| Invention | Cutinase variant | 4.5 |
| Reference | Parent cutinase | 0.3 |
| | None | 0.1 |

The results show that the variant is effective in solubilizing polyester.

In another experiment, the cutinase variant was tested for 2 hours at 65°C with and without the addition of a non-ionic surfactant (alcohol ethoxylate, product name Softanol 50), using various amounts of the variant from 0.5 to 200 LU/ml. The results showed more solubilization in the presence of non-ionic surfactant.

### Example 9: Hydrolysis of polycaprolactone and polyester film

About 0.1 g of polycaprolactone or polyester film were put in tubes. They were soaked in 5ml of 50mM GlyGly buffer (pH 8.5) with or without a variant of *H*. *insolens* cutinase (450 LU). They were incubated at 70°C for 5 hours. After the reaction we observed a thin layer of hydrolysate on the surface of the tubes with enzyme, both with polycaprolactone and with polyester film. On the other hand no change was observed in controls without enzyme. In the case of polycaprolactone there was 10% of weight loss. We see no weight change of polyester.

### Example 10: cPET hydrolysis

The performance of a cutinase variant was compared with the parent enzyme (*H. insolens* cutinase). The trials were done as follows:

An oligomer-stained swatch of (black) PET-fabric (app. 4cm x 13cm) is subjected to the enzyme-treatment at relatively low agitation in a so-called mini-tergitometer apparatus. The PET-fabric is mounted onto a cylindrical, perforated holder (radius ca.2 cm, height ca 6 cm) , that rotates around its axis, and with the oligomer stained side of the PET fabric facing the exterior of the cylinder.

The fabric is immersed in a 150ml glass-beaker containing 100ml of the treatment solution at a given temperature (here 65°C). After a given treatment time (here 90minutes) the PET swatch is removed from the bath and rinsed in deionized water and air dried.

After conditioning the swatches are visually ranked (with respect to oligomer stain removal) on the side having the oligomer-staining. The rating being as follows:

| | |
|---|---|
| -2: | Sample significantly worse than blank (no enzyme) |
| -1: | Sample slightly worse than blank (no enzyme) |
| 0: | Sample can not be distinguished from blank |
| 1: | Sample slightly improved vs blank |
| 2: | Sample significantly improved over blank |

Also, the swatches are read spectrofotometrically (apparatus: Hunterlab Reflectometer) to quantify the color strength (K/S-value at 600nm).

The table below summarizes the test-conditions for a trial comparing the performance the enzymes under similar conditions:

| | |
|---|---|
| Temperature: | 65°C |
| Buffer/pH: | 50 mM glycine buffer, pH 10.3 |
| Treatment time (min) | 90 |
| Dosage of Enzyme (LU/g) | 30000 |

Results from the trial are summarized below

| Enzyme | Visual rating (avg.) | K/S Difference |
|---|---|---|
| | | @ 600 nm |
| None | 0 (defined) | 2.33 |
| Parent cutinase | 0 | 2.38 |
| Cutinase variant | 1.5-2.0 | 2.89 |

From this set of experiments it thus appears that the parent enzyme provides no or only very limited effect at the given test conditions (probably because the temperature is too high for the enzyme to retain activity), while the cutinase variant provides a substantial removal of the oligomer staining from the PET-fabric.

### Example 11: cPET hydrolysis

The pH and temperature profile of a variant of *H. insolens* cutinase was tested in a model disperse dyeing experiment. The trials were performed as follows:

An oligomer-stained swatch of (black) PET-fabric is subjected to the conditions of a typical disperse dyeing sequence in a Werner Mathis Labomat. In overview of the process, the swatch is added to a buffer solution, heated to 130°C, cooled down to the treatment temperature. Enzyme or buffer is added and then held at the desired temperature for 30 minutes. The solution is cooled down to room temperature and turbidity in the wash liquor is measured. The reduction in turbidity is a direct measure of the cutinase activity, corresponding to hydrolyzed cPET oligomers.

### Detailed description of the experiment:

A black PET (app. 4cm x 13cm) swatch is added 140 ml 100 mM Britton-Robinson buffer containing 0.2 g/l Lutensol AT11 (BASF) and loaded in the Labomat (32 rotation per minute).

The Labomat is heated to 130°C at a gradient of 9°C/minute, and held for 10 minutes.

The beakers are cooled to run temperature (according to table below) at a gradient of 9°C/minute, and held for 1 minute.

10 mL enzyme solution (100 LU/ml of the variant) or buffer solution (0 LU/ml) at appropriate pH is injected to the beakers.

The Labomat is re-heated to temperature at a gradient of 2°C/minute, and held for 30 minutes.

The swatches are removed, and the wash liquor is cooled down to room temperature.

Turbidity of the wash liquors are measured.

Evaluation: Turbidity is measured on Hach 18900 Ratio Turbidimeter (standardized with 1.8, 18, and 180 NTU Turbidity Standards). Enzyme performance is calculated relative to a blank as the difference between turbidity of blank liquor (no enzyme) and turbidity of enzyme treated liquor.

The relative performance (reduction in turbidity) of the cutinase variant is calculated, and the results are shown in the following table. When a negative number is obtained, then the result is given as "negative". A negative number is assumed to be an artifact, caused by the variation of the set up.

| Temperature | pH 7 | pH 8 | pH 9 | pH 10 |
|---|---|---|---|---|
| 60°C | 39 | 57 | 37 | 14 |
| 65 °C | 39 | 16 | 60 | 30 |
| 70°C | 25 | 12 | 54 | 33 |
| 75°C | 22 | 50 | 114 | 58 |
| 85 °C | negative | negative | 15 | negative |

The results show that the cutinase variant is active over a broad pH and temperature range, with optimum oligomer removal in the current set up around pH 9 and 75°C. Inactivation seems to occur at or above 85°C.

### Example 12: cPET hydrolysis

The effect of treatment time was investigated for a variant of *H. insolens* cutinase in a model disperse dyeing experiment. The trials were performed as follows:

An oligomer-stained swatch of (black) PET-fabric is subjected to the conditions of a typical disperse dyeing sequence in a Werner Mathis Labomat. In overview of the process, the swatch is added to a buffer solution, heated to 130°C, cooled down to the treatment temperature. Enzyme or buffer (100 mM Britton-Robinson pH 9) is added, and then held at 75°C for 0-40 minutes. The solution is cooled down to room temperature and turbidity in the wash liquor is measured. The reduction in turbidity is a direct measure of the cutinase activity, corresponding to hydrolyzed cPET oligomers.

### Detailed description of the experiment:

A black PET (app. 4cm x 13cm) swatch is added to 140 ml 100 mM Britton-Robinson buffer containing 0.2 g/l Lutensol AT11 (BASF) and loaded in the Labomat (32 rotation per minute).

The Labomat is heated to 130°C at a gradient of 9°C/minute, and the temperature is held for 10 minutes.

The beakers are cooled to 75°C at a gradient of 9°C/minute, and held for 1 minute.

10 mL enzyme solution (100 LU/ml of variant) or 100 mM Britton-Robinson buffer pH 9.0 (0 LU/ml) is injected into the beakers.

The Labomat is re-heated to 75°C at a gradient of 2°C/minute, and held for the appropriate number of minutes (0-40 minutes, see table below).

The swatches are removed, and the wash liquor is cooled down to room temperature.

Turbidity of the wash liquors are measured.

Evaluation: Turbidity is measured on Hach 18900 Ratio Turbidimeter (standardized with 1.8, 18, and 180 NTU Turbidity Standards). Enzyme performance is calculated relative to a blank at time equal to zero: Turbidity of blank liquor at time zero (no enzyme) subtracted turbidity of enzyme treated liquor (at a given time).

The relative performance (reduction in turbidity) of the cutinase variant was calculated, and the results are shown in the following table.

| Time (minutes) | Relative performance (Reduction in turbidity) |
|---|---|
| 0 | 0 |
| 5 | 42 |
| 10 | 48 |
| 15 | 62 |
| 20 | 69 |
| 25 | 85 |
| 30 | 72 |
| 40 | 78 |

The results show that the effect of the enzyme is increased over time. At the current enzyme dose and oligomer concentration, it seems to level off above approx. 20 minutes.

### Example 13: Fiber modification

The effect on wetting characteristics of a disperse dyed polyester fabric was investigated by treating the fabric with a variant of *H. insolens* cutinase prior to dyeing. The experiment therefore consisted of two phases, the actual fiber modification and the disperse dyeing procedure.

### Phase 1 - Fiber Modification:

| | |
|---|---|
| Equipment: | Atlas Launder-O-meter LP2 |
| Fabric: | knit 100 % scoured polyester from Testfabrics |
| pH: | 50 mM potassium phosphate buffer, pH 7 |
| Abrasives: | 5 big steel balls |
| Beaker Vol.: | 120 mL |
| Treatment: | 2 hours 65°C then ramped up to 90°C and held for 1 hour |
| Swatch Prep: | Cut 3* 1.5 g swatch of fabric, 3 per beaker = 4.5 g |

### Rinse:

Rinse in deionized water.

### Phase 2 - Dyeing - disperse dye:

### Dye Solution:

Add together with deionized water to make liquor ratio 1:20-
0.4 % Dianix Red (DyStar) SE-CB (owf)
pH to 4.5 - 5

### Dyeing Procedure:

1. One swatch per treatment from the fiber modification is used for the dyeing (1.5 g/swatch is used for the liquor ratio calculation).
2. Make dyebath according to the recipe above. Add the cold dye solution to the Labomat beakers and heat to 55°C at a gradient of 3.5°C/minute. Run for 5 minutes once temperature has been reached.
3. Add the fabric to the beaker.
4. Raise temperature to 130°C at a gradient of 1.5°C/minute. Dye for 30 minutes.
5. Cool to 70°C at a gradient of 5°C/minute. Drop bath, but collect, and rinse fabric hot (60°C) for 10 minutes. Follow the hot rinse with a room temperature overflow rinse until all bleeding had stopped.
6. Let air dry overnight.

### Tests/Analysis:

AATCC Test Method 61 - Colorfastness to washing
Percent Dyebath Exhaustion - Spectrophotometer
K/S and L* - Reflectometer
AATCC TM-79 Drop Test

### Results:

The results from the fiber modification are shown in the following table.

| Variant dosage | Staining (AATCC TM-61) | Color Change (K/S @ 530 before and after TM-61) | Drop Test (AATCC TM-79) |
|---|---|---|---|
| Blank | 4.5 | 5 | 53 sec. |
| 50 LU/mL | 4.5 | 5 | 18 sec. |
| 100 LU/mL | 4.5 | 5 | 15 sec. |

The results show that the treatment of polyester with the variant increases the wetting substantially. No adverse effects are noticed on the dyeability with the disperse dye in the current set-up.

### Example 14: Malodor reduction in textiles soiled with human sweat/sebum by use of a cutinase variant in laundry

The performance of cutinase, with respect to malodor reduction, can be tested in a one-cycle washing trial carried out in a Terg-O-tometer.

### Experimental conditions:

Washing liquor: 1000 ml per beaker
Swatches: 100 % polyester (interlock knitted, previously cleaned by Soxhlet extraction). 24 swatches (3.3 x 3.5 cm) per beaker.
Soil: Human male axillary sweat and sebum applied by wiping the armpits after exercise.
Detergent: 5 g/L of a standard color detergent. No pH adjustment.
Water hardness: 3.2 mM Ca²⁺/Mg²⁺ (in a ratio of 5:1)
Wash Temperature: 30°C
Wash time: 30 min
Rinse: 15 minutes in running tap water

### Evaluation:

After wash the wet swatches are placed in closed, tinted 200 ml glasses. A trained sensory panel (9-11 judges) evaluates the odor by sniffing the headspace over the wet samples and evaluates the total odor intensity. The odor intensity is noted by placing a mark on an unstructured line scale measuring 15 cm, with word anchors at each end ('nothing' at the beginning of the scale and 'very strong' at the end). All evaluations are performed twice. The swatches are evaluated on day 1, 2 and 3 after wash (swatches are kept in the glasses at all times).

## Claims

1. A variant of a parent cutinase, wherein:
a) the parent cutinase is the cutinase of *H. insolens* strain DSM 1800,
b) the variant has an amino acid sequence having 1-20 alterations compared to the parent cutinase,
c) the variant comprises substitution of a negative amino acid residue at a position corresponding to position E6, E10, E30, E47 D63, E82 and/or E179 by a neutral or positive amino acid or a substitution with Pro at a position corresponding to A14 or R51 in the parent cutinase,
d) the variant has hydrolytic activity towards cyclic tri(ethylene terephthalate) or terephthalic acid bis(2-hydroxyethyl)ester dibenzoate, and
e) the variant is more thermostable than the parent cutinase.

2. The variant of the preceding claim which comprises a substitution corresponding to E6N/Q, E10N/Q, E47K/R and/or E179N/Q (*H. insolens* cutinase numbering).

3. The variant of either preceding claim which comprises a substitution corresponding to A14P or R51 P in the cutinase of *Humicola insolens* strain DSM 1800.

4. The variant of any preceding claim which has one, two, three, four, five or six of said substitutions.

5. The variant of any preceding claim which has substitutions corresponding to one of the following in the cutinase of *Humicola insolens* strain DSM 1800:
a) R51 P
b) E6N/Q + L1381
c) A14P + E47K
d) E47K
e) E179N/Q
f) E6N/Q + E47K + R51P
g) A14P + E47K + E179N/Q
h) E47K + E179N/Q
i) E47K + D63N
j) E6N/Q + A14P + E47K + R51P + E179N/Q
k) E6N/Q + E10N/Q + A14P + E47K + R51 P + E179N/Q, or
l) Q1P + L2V + S11C + N15T + F24Y + L461 + E47K

6. The variant of any preceding claim which has a denaturation temperature which is at least 5° higher than the parent cutinase, when measured at pH 8.5

7. A DNA sequence encoding the variant of any preceding claim.

8. A vector comprising the DNA sequence of the preceding claim.

9. A transformed host cell harboring the DNA sequence of claim 7 or the vector of claim 8.

10. A method of producing the variant of any of claims 1-6 comprising
a) cultivating the cell of the preceding claim so as to express and preferably secrete the variant, and
b) recovering the variant.

11. A process for enzymatic hydrolysis of a cyclic oligomer of poly(ethylene terephthalate), which process comprises treating the cyclic oligomer with the cutinase variant of any of claims 1-6.

12. The process of the preceding claim, in which the cyclic oligomer is cyclic tri(ethylene terephthalate).

13. The process of claim 11 or 12 wherein the treatment is done at 60-80°C, preferably at 65-75°C.

14. The process of any of claims 11-13 wherein the cyclic oligomer is present in and on the fibers of a polyester containing fabric or yarn.

15. The process of any of claims 11-14 which further comprises subsequently rinsing the fabric or yarn, preferably rinsing with an aqueous solution having a pH in the range of from about pH 7 to about pH 11.

16. A process for dyeing polyester fabric or yarn, comprising:
a) treating the fabric or yarn with the cutinase variant of any of claims 1-6, and
b) dyeing the treated fabric with a reactive dye or a disperse dye.

17. A detergent composition comprising a surfactant and the variant of any of claims 1-6.

18. A process for improving the functional finish of a PET-containing yarn or fabric comprising
a) treating the yarn or fabric with the variant of any of claims 1-6, and
b) subsequently treating the yarn or fabric with a finishing agent selected from the group consisting of softeners, anti-crease resins, anti-static agents, anti-soiling agents.

## Patentansprüche

1. Variante einer Eltern-Cutinase, wobei:
a) die Eltern-Cutinase die Cutinase von *H. insolens,* Stamm DSM 1800, ist,
b) die Variante eine Aminosäuresequenz mit 1-20 Änderungen im Vergleich zu der Eltern-Cutinase aufweist,
c) die Variante eine Substitution eines negativen Aminosäurerestes an einer Position entsprechend Position E6, E10, E30, E47 D63, E82 und/oder E179 mit einer neutralen oder positiven Aminosäure oder eine Substitution mit Pro an einer Position entsprechend A14 oder R51 in der Eltern-Cutinase umfasst,
d) die Variante hydrolytische Aktivität gegenüber cyclischem Tri(ethylenterephthalat) oder Terephthalsäure-bis(2-hydroxyethyl)esterdibenzoat aufweist, und
e) die Variante wärmestabiler ist als die Eltern-Cutinase.

2. Variante des vorhergehenden Anspruchs, die eine Substitution entsprechend E6N/Q, E10N/Q, E47K/R und/oder E179N/Q (*H*. *insolens-*Cutinase-Nummerierung) umfasst.

3. Variante nach einem der beiden vorhergehenden Ansprüche, die in der Cutinase von *Humicola insolens,* Stamm DSM 1800, eine Substitution entsprechend A14P oder R51P umfasst.

4. Variante nach einem vorhergehenden Anspruch, die eine, zwei, drei, vier, fünf oder sechs der Substitutionen aufweist.

5. Variante nach einem vorhergehenden Anspruch, die in der Cutinase von *Humicola insolens,* Stamm DSM 1800, Substitutionen aufweist, die einer der folgenden entsprechen:
a) R51P
b) E6N/Q + L138I
c) A14P + E47K
d) E47K
e) E179N/Q
f) E6N/Q + E47K + R51P
g) A14P + E47K + E179N/Q
h) E47K+E179N/Q
i) E47K + D63N
j) E6N/Q + A14P + E47K + R51P + E179N/Q
k) E6N/Q + E10N/Q + A14P + E47K + R51P + E179N/Q oder
l) Q1P+L2V+S11C+N15T+F24Y+L46I+E47K

6. Variante nach einem vorhergehenden Anspruch, die eine Denaturierungstemperatur aufweist, die mindestens 5 ° höher ist als diejenige der Eltern-Cutinase, gemessen bei pH 8,5.

7. DNA-Sequenz, die die Variante nach einem vorhergehenden Anspruch codiert.

8. Vektor, der die DNA-Sequenz des vorhergehenden Anspruchs einschließt.

9. Transformierte Wirtszelle, die die DNA-Sequenz nach Anspruch 7 oder den Vektor nach Anspruch 8 beherbergt.

10. Verfahren zur Herstellung der Variante nach einem der Ansprüche 1 - 6, umfassend
a) Kultivieren der Zelle des vorhergehenden Anspruchs, so dass die Variante exprimiert und vorzugsweise sezerniert wird, und
b) Gewinnen der Variante.

11. Verfahren zur enzymatischen Hydrolyse eines cyclischen Oligomers von Poly(ethylenterephthalat), wobei das Verfahren die Behandlung des cyclischen Oligomers mit der Cutinase-Variante nach einem der Ansprüche 1-6 umfasst.

12. Verfahren des vorhergehenden Anspruchs, wobei das cyclische Oligomer cyclisches Tri(ethylenterephthalat) ist.

13. Verfahren nach Anspruch 11 oder 12, wobei die Behandlung bei 60 - 80 °C, vorzugsweise bei 65 - 75 °C vorgenommen wird.

14. Verfahren nach einem der Ansprüche 11 - 13, wobei das cyclische Oligomer in und auf den Fasern eines Polyester-enthaltenden Textilstoffs oder Garns vorhanden ist.

15. Verfahren nach einem der Ansprüche 11 - 14, welches weiterhin anschließend das Spülen des Textilstoffs oder Garns, vorzugsweise das Spülen mit einer wässrigen Lösung mit einem pH-Wert im Bereich von etwa pH 7 bis etwa pH 11 umfasst.

16. Verfahren zum Färben von Polyester-Textilstoff oder -Garn, umfassend:
a) Behandeln des Textilstoffs oder Garns mit der Cutinase-Variante nach einem der Ansprüche 1 - 6,
und
b) Färben des behandelten Stoffs mit einem Reaktivfarbstoff oder einem Dispersionsfarbstoff.

17. Detergenszusammensetzung, umfassend ein oberflächenaktives Mittel und die Variante nach einem der Ansprüche 1 - 6.

18. Verfahren zur Verbesserung der funktionellen Ausrüstung eines PET-enthaltenden Garns oder Textilstoffs, umfassend
a) Behandeln des Garns oder Textilstoffs mit der Variante nach einem der Ansprüche 1 - 6, und
b) anschließendes Behandeln des Garns oder Textilstoffs mit einem Veredlungsmittel, ausgewählt aus der Gruppe, bestehend aus Weichmachern, Antiknitterharzen, antistatischen Mitteln, schmutzabweisenden Mitteln.

## Revendications

1. Variant d'une cutinase parente, dans lequel :
a) la cutinase parente est la cutinase de la souche DSM 1800 de *H. insolens,*
b) le variant a une séquence en acides aminés ayant 1-20 altérations par rapport à la cutinase parente,
c) le variant comprend une substitution d'un résidu d'acide aminé négatif à une position correspondant à la position E6, E10, E30, E47, D63, E82 et/ou E179 par un acide aminé neutre ou positif, ou une substitution par Pro à une position correspondant à A14 ou R51 dans la cutinase parente,
d) le variant possède une activité hydrolytique vis-à-vis du tri(éthylène téréphtalate) cyclique ou de l'ester dibenzoïque de l'acide bis(2-hydroxyéthyl) téréphtalique, et
e) le variant est plus thermostable que la cutinase parente.

2. Variant selon la revendication précédente, qui comprend une substitution correspondant à E6N/Q, E10N/Q, E47K/R et/ou E179N/Q (numérotation selon la cutinase de *H insolens).*

3. Variant selon l'une quelconque des revendications précédentes, qui comprend une substitution correspondant à A14P ou R51P dans la cutinase de *Humicola insolens,* souche DSM 1800.

4. Variant selon l'une quelconque des revendications précédentes, qui possède une, deux, trois, quatre, cinq ou six desdites substitutions.

5. Variant selon l'une quelconque des revendications précédentes, qui possède des substitutions correspondant à l'une des suivantes dans la cutinase de *Humicola insolens,* souche DSM 1800 :
a) R51P
b) E6N/Q+L1381
c) A14P + E47K
d) E47K
e) E179N/Q
f) E6N/Q + E47K + R51P
g) A14P + E47K + E179N/Q
h) E47K + E179N/Q
i) E47K + D63N
j) E6N/Q + A14P + E47K + R51P + E179N/Q
k) E6N/Q + E10N/Q + A14P + E47K + R51P + E179N/Q, ou 1)Q1P+L2V+S11C+N15T+F24Y+L46I+E47K.

6. Variant selon l'une quelconque des revendications précédentes, qui possède une température de dénaturation qui est au moins 5° supérieure à celle de la cutinase parente, lorsque mesurée à pH 8,5.

7. Séquence d'ADN codant le variant selon l'une quelconque des revendications précédentes.

8. Vecteur comprenant la séquence d'ADN de la revendication précédente.

9. Cellule hôte transformée portant la& séquence d'ADN de la revendication 7 ou le vecteur de la revendication 8.

10. Procédé pour produire le variant selon l'une des revendications 1-6, comprenant :
a) la culture de la cellule selon la revendication précédente de manière à exprimer et, de préférence, sécréter le variant, et
b) la récupération du variant.

11. Procédé pour l'hydrolyse enzymatique d'un oligomère cyclique de poly(éthylène téréphtalate), ledit procédé comprenant le traitement de l'oligomère cyclique avec le variant de cutinase de l'une des revendications 1-6.

12. Procédé selon la revendication précédente, dans lequel l'oligomère cyclique est le tri(éthylène téréphtalate) cyclique.

13. Procédé selon la revendication 11 ou 12, dans lequel le traitement est réalisé à 60-80°C, de préférence à 65-75°C.

14. Procédé selon l'une des revendications 11-13, dans lequel l'oligomère cyclique est présent dans et sur les fibres d'un tissu ou fil contenant du polyester.

15. Procédé selon l'une des revendications 11-14, qui comprend en outre le rinçage ultérieur du tissu ou fil, de préférence le rinçage avec une solution aqueuse ayant un pH compris dans la gamme de environ pH 7 à environ pH 11.

16. Procédé pour colorer un tissu ou fil en polyester, comprenant :
a) le traitement du tissu ou fil avec le variant de cutinase de l'une des revendications 1-6, et
b) la coloration du tissu traité avec un colorant réactif ou un colorant dispersé.

17. Composition détergente comprenant un tensio-actif et le variant de l'une des revendications 1-6.

18. Procédé pour améliorer la finition fonctionnelle d'un tissu ou fil contenant du PET, comprenant :
a) le traitement du tissu ou fil avec le variant de l'une des revendications 1-6, et
b) le traitement subséquent du tissu ou fil avec un agent de finition choisi dans le groupe constitué des plastifiants, résines anti-créases, agents anti-statiques, agents anti-dépôt.
